# EUROPEAN PATENT APPLICATION

(11) **EP 1 394 219 A1**
(43) Date of publication of application: **03.03.2004**
(21) Application number: 03022839.9
(22) Date of filing: 08.06.2000
(51) Int. Cl.: C09B 23/02, G01N 31/22

(54) **PH sensitive cyanine dyes as reactive fluorescent reagents**

(30) Priority: 09.06.1999 US 138297 P
(62) Divisional of application: 00942696.6
(71) Applicant: CARNEGIE MELLON UNIVERSITY, Pittsburgh Pennsylvania 15213 (US); Amersham Pharmacia Biotech UK Limited, Little Chalfont, Buckinghamshire HP7 9NA (GB)
(72) Inventor: Mujumdar, Ratnakar, Glenshaw, PA 15116 (US); Smith, John Anthony, Cardiff CF14 6JS (GB)
(74) Representative: Franks, Barry Gerard

(57) **Abstract**

The present invention provides pH sensitive cyanine dyes having the structure (I) 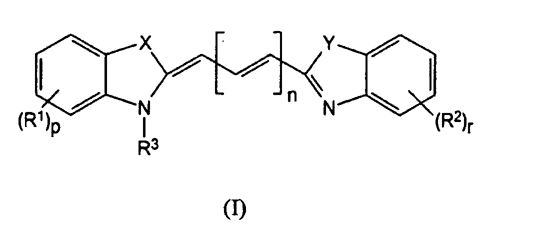 wherein X and Y are independently selected from >C(C₁ - C₄alkyl)₂, sulfur and oxygen; R¹ and R² are independently selected from H, CH₂NH₂, SO₃⁻, phosphate, phosphonate, quaternary ammonium, (CH₂)_{q}COOH, NCS, CH₂NH-COR⁷, where R⁷ is C₁ - C₂₀ straight or branched alkyl and (CH₂)_{q}COOH where q is an integer from 0 - 10; R³ is H or -L-P where L is selected from C₁ - C₂₀ straight or branched alkyl optionally containing 0, 1 or 2 unsaturated groups selected from alkenyl and alkynyl, and P is selected from a reactive group, H, C₁ - C₂₀ straight or branched alkyl, SO₃⁻, NH₂, quaternary ammonium, CH₂NH-COR⁸, where R⁸ is C₁ - C₂₀ straight or branched alkyl, -(CH₂)ₘCOOH, NHR⁹ where R⁹ is C₁ - C₂₀ straight or branched alkyl; n is an integer from 0 - 3; p and r are independently 0, 1, 2, 3 or 4 and where p and/or r is greater than 1, each R¹ and each R² may be different, and m is an integer from 1 - 10; and salts and protonated derivatives thereof and methods of using same as fluorescent reagents.

## Description

The present application claims benefit to U.S. Provisional Application No. 60/138,297, filed June 9, 1999, the entire contents of which is hereby incorporated, in its entirety, by reference.

The present invention provides pH sensitive cyanine dyes and methods of using them as fluorescent reagents.

Cyanine and related polymethine dyes having light absorbing properties have been employed in the photographic films, optical recording media, and recently as luminescence (fluorescent and phosphorescent) dyes for biological investigations. Several review articles have been appeared on the syntheses and applications of cyanine dyes, F. Hamer "Cyanine Dyes and Related Compounds", A. Weissberger, Ed., Interscience Publishers, New York, (1964); D. M. Sturmer "Synthesis and Properties - Cyanine and Related Dyes," The Chemistry of Heteroaromatic Compounds, Vol. 30A. Weissberger and E. C. Taylor Eds., John Wiley & Son, New York, NY, (1977); G. E. Ficken "The Chemistry of Cyanine Dyes," Vol. 4, pp 211, K. Venkataraman, Ed., Academic Press, (1971); K. Venkataram "Cyanine Dyes", The Chemistry of Synthetic Dyes, Vol. 2, pp 1143, L. Ventataraman, Ed. Academic Press, 19xx; D. J. Fry "Cyanine Dyes and Related Compounds" Rodds' Chemistry of Carbon Compounds, Vol. IV, Ed. M. J. Ansell, pp 267-296, 1985.

A typical cyanine may be defined as a molecule having two nitrogen containing heteroaromatic rings (A and A') joined by a polymethine chain as shown in formula 1. Groups R₁ and R₂ are essentially alkyl or substituted alkyl groups and "m" is an integer selected from the group consisting of 0, 1, 2, 3, and 4. These dyes have cationic character due to delocalized positive charge on the chromophore. This delocalization also accounts for very high extinction coefficients of cyanine dyes.

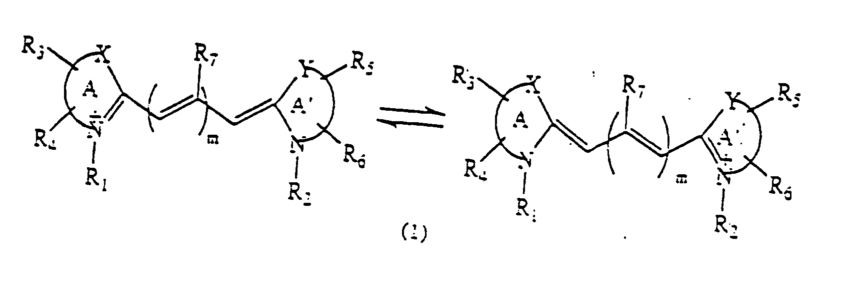

Wherein,
Groups R₁ and R₂ are alkyl or substituted alkyl groups,
Groups R₃, R₄, R₅, R₆ and R₇ are H, water solubilizing groups,
halogens, alkoxy, hydroxy, amine, amide, carboxylic acid, or
carboxylic ester.

Typically rings "A" and "A"' are selected from the following heteroaromatic rings. 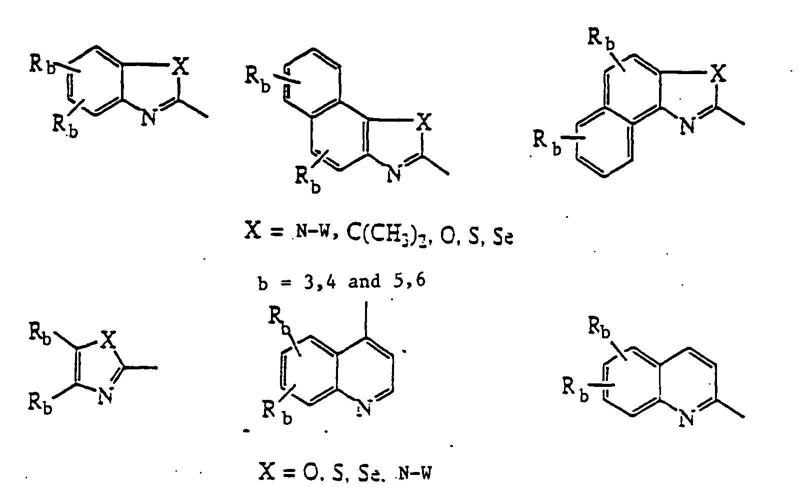
Where R₃, R₄, R₅, R₆ and X are hereinbefore defined. W is selected from hydrogen,
   -(CH₂)ₙR, where n is an integer from 1 to 26 and R is selected from hydrogen, amino, aldehyde, acetal, ketal, halo, cyano, aryl, heteroaryl, sulphonate, sulphate, carboxylate, substituted amino, nitro, primary amide, substituted amide and groups reactive with amino, hydroxyl, carbonyl, phosphoryl and sulphydryl groups.

The following are examples of known pH sensitive cyanine dyes.

The spectral properties of the cyanine dyes are only slightly sensitive to solvent changes and they are generally considered as pH insensitive dyes. However, only a few cyanines are known to show pH dependency. Amine or carboxylic acid groups directly attached to the aromatic ring of the heteroaromatic system affect the cation delocalization between the nitrogens. Absorption bands of amino-cyanine dyes (formula 2) are very broad with almost total mixing of main electronic transition and vibronic side bands at neutral pH. In a mildly acidic solutions or when the amine is blocked by an acetyl group the transition becomes sharper, more intense and shifts to the blue. These amino-cyanines are virtually non-fluorescent in neutral solution (R. B. Mujumdar, L. E. Ernst, S.R. Mujumdar, and A. S. Waggoner, "Cyanine Dye Labeling Reagents containing Isothiocyanate Groups", Cytometry vol. 10, pp 11-19 (1989)).

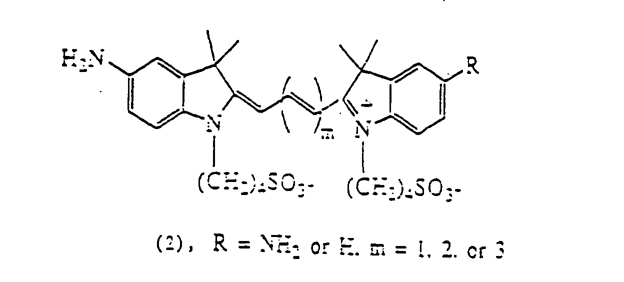

Squaraines usually are pH sensitive but its derivatives (formula 3) are insensitive in a biological pH range (pH 6-pH 9) and therefore, can not be used effectively as pH sensitive dyes for biological investigations (E. Terpetsching, H. Szmacinski, A. Ozinskas, and J. R. Lakowicz, "Synthesis of squaraine-N-Hydroxysuccinimide Esters and Their Biological Application as Long-Wavelength Fluorescent Labeling", Analytical Biochemistry vol. 217, pp 197-204 (1994)).

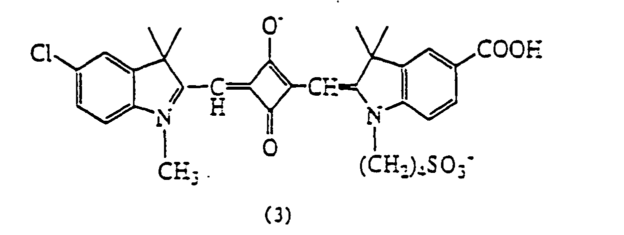

Kolesnikov and Povolotskii (1986) have studied ¹H NMR of a simple mono N-alkylated cyanine (formula 4), but its synthesis and spectral properties (absorbance, emission) are not discussed (A. M. Kolesnikov, and M. I. Provolotskii, Teor. Eksp. Khim, vol. 24, pp 225-227 (1986)). Brooker *et*. *al* (1940) have reported synthesis of monoalkylated cyanines (Formula 5). These compounds (called anhydronium bases) are unstable and fade badly even when trying to measure their absorbance (L. G. S. Brooker, R. H. Sprague, C. P. Smyth, and G. L. Lewis. "Halochromism of Anhydronium Bases Related to the Cyanine Dyes", J. Chem. Soc., Vol. 62, pp 1116-1124 (1940)). Zubaroviskil (1972) has described the synthesis of thiacarbocyanine (Formula 5), but its spectral properties are not discussed (V. M. Zubarvotskii "Synthesis of thiacarbocyanines without alkyls at the nitrogen atoms of their benzothiazole nuclei", Khim. Geterotsikl. Soedi., Vol. 11, pp 1579-80 (1972)). 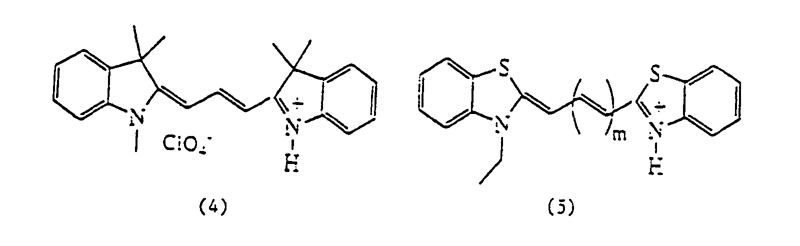 The dyes of formula 4 and 5 have an exchangeable hydrogen on one of the nitrogens, but they are water insoluble, lack reactive groups and are unstable, rendering them unsuitable for biological applications.

Fluorescent dyes are generally known and used for fluorescence labeling and detection of various biological and non-biological materials by procedures such as fluorescence microscopy, fluorescence immunology and flow cytometry. Fluorescent dyes may also be used as tracers to measure, for example, intracellular and extracellular ionic concentrations simultaneously. In this way ionic gradients across membranes may be measured. Moreover, it is possible to monitor two ions simultaneously using two different fluorescent probes.

Four commonly used classes of fluorescent dyes are those based on fluorescein (green fluorescence) and rhodamine (orange fluorescence), coumarin and pyrene (blue fluorescence) chromophores. Commonly available fluorescent pH indicators are generally either fluorescein or pyrene derivatives. Dyes based on fluorescein have a number of disadvantages, including their tendency to photobleach when illuminated with strong excitation sources. The resulting rapid loss of image with time makes detection and quantitation more difficult. Fluorescein derivatives also have a pH sensitive absorption spectrum and fluorescence yield decreases markedly below pH 8. Pyrene derivative, such as pyrene trisulphonates have broad absorption and emission spectra and relatively low extinction coefficients. A wide array of fluorescent pH indicators are available from Molecular Probes, Portland OR and information relating to same is described in "Molecular Probes - Handbook of Fluorescent Probes and Research Chemicals (Ed. R.P. Haugland (1996)). Most of the fluorescent pH indicators described therein fluorescence only in neutral or alkaline solution.

There are five dyes available from Molecular Probes which are suggested for acidic pH environments. These include dyes referred to as LYSOSENSOR probes which are weak bases that become more fluorescent in acidic environments. Among these is LYSOSENSOR YELLOW/BLUE which has the following formula 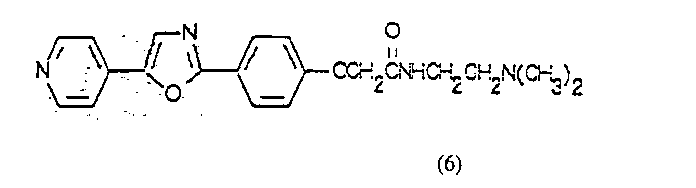 and is reported to undergo a pH dependent emission shift to longer wavelengths in acidic environments when illuminated near its excitation isosbestic point (~360nm), as well as a pH-dependent excitation shift when detected near its emission isosbestic point (~490). These changes require ratiometric pH measurement in acidic organelles or other acidic environments.

Other LYSOSENSOR pH sensitive fluorescent indicators include LYSOSENSOR BLUE DND-167 which is non-fluorescent in non-acidic environments, soluble in DMSO, has a pKa of 5.1, fluoresces at pH of 4.5-6 with an emission wavelength of 425 nm when excited at 373 nm and has the following structure

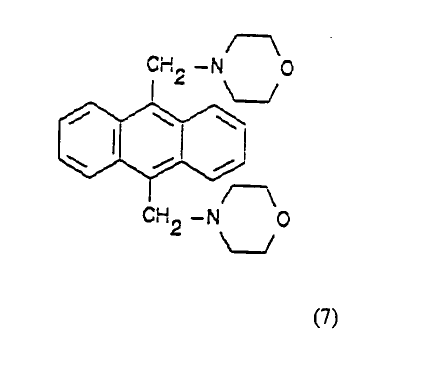

LYSOSENSOR GREEN DND-189 is also non-fluorescent in non-acidic environments, soluble in DMSO, has a pKa of 5.2, fluoresces at a pH of 4.5-6 with an emission wavelength of 505 nm when excited at 443 nm and has the following structure

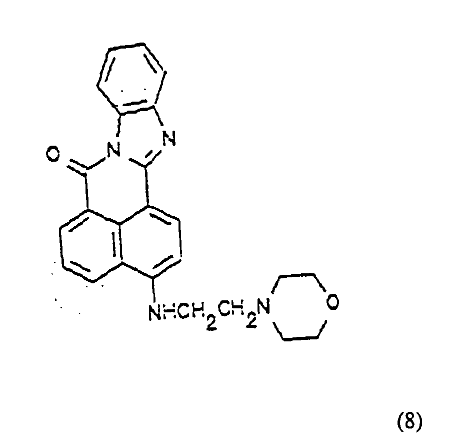

LYSOSENSOR BLUE DND-192 fluoresces brightly at neutral pH, is soluble in DMSO, has a pKa of 7.5, fluoresces at a pH of 6.5-8 with an emission wavelength of 425 nm when excited at 374 nm and has the following structure

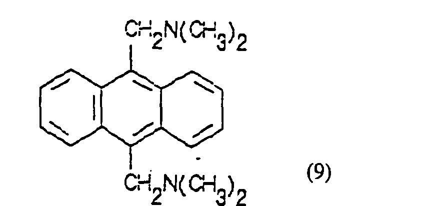

LYSOSENSOR GREEN DND-153 also fluoresces brightly at neutral pH, is soluble n DMSO, has pKa of 7.5, fluoresces at a pH of 6.5-8 with an emission wavelength of 505 nm when excited at 442 nm and has the following structure

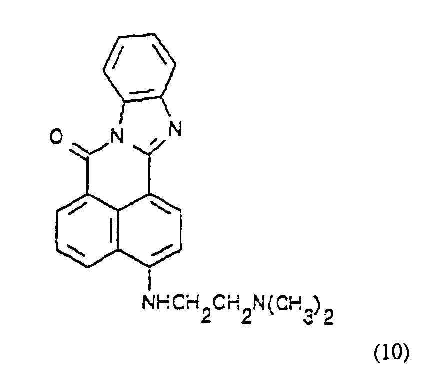

It is noted that because the LYSOSENSOR probes may localize in membranes rather than in an aqueous medium, it is probable that the pKa values noted above will be different in a cellular environment and that qualitative or semi-qualitative comparisons of cellular pH may be possible.

Another group of compounds available from Molecular Probes for fluorescent pH measurement are fluorescein derivatives wherein an electron-withdrawing group has been introduced to lower the pKa of the phenolic group to 5 or below. The product literature reports that fluorinated fluorescein dyes are still pH sensitive in moderately acidic solutions, with pKa values of about 4.7.

The final group of compounds available from Molecular Probes for fluorescent pH monitoring are rhodol derivatives which are structural hybrids of fluorescein and rhodamine dyes which have retained the pH-sensitive properties conferred by a phenolic hydroxy group while also containing an amine substituent to reduce the pKa values. Among these dyes is C-NERF which has the following structure 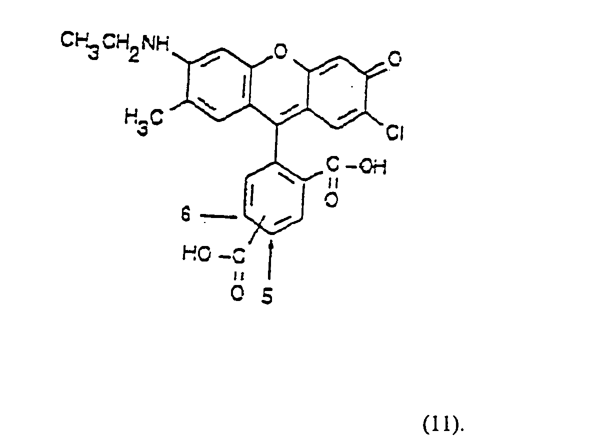 This dye permits ratiometric pH measurements using two principal visible lines of an argon-ion laser (514 nm and 488 nm) with emission reported at 540 nm.

While there are a small number of compounds which fluoresce in acidic pH conditions there is a need for water soluble, non-fluorescein derivatives which may be used as acidic pH indicators which fluoresce in the 400-800 nm region, such as is provided by the present invention. The cyanines described in this invention preferably have functional groups to enhance aqueous solubility. The cyanines preferably have at least one sulfonate group which may be an aryl sulfonate or aralkyl sulfonate. Alternatively, the water solubilizing group of this invention may be a phosphonate or a phosphate.

### BRIEF DESCRIPTION OF THE DRAWINGS

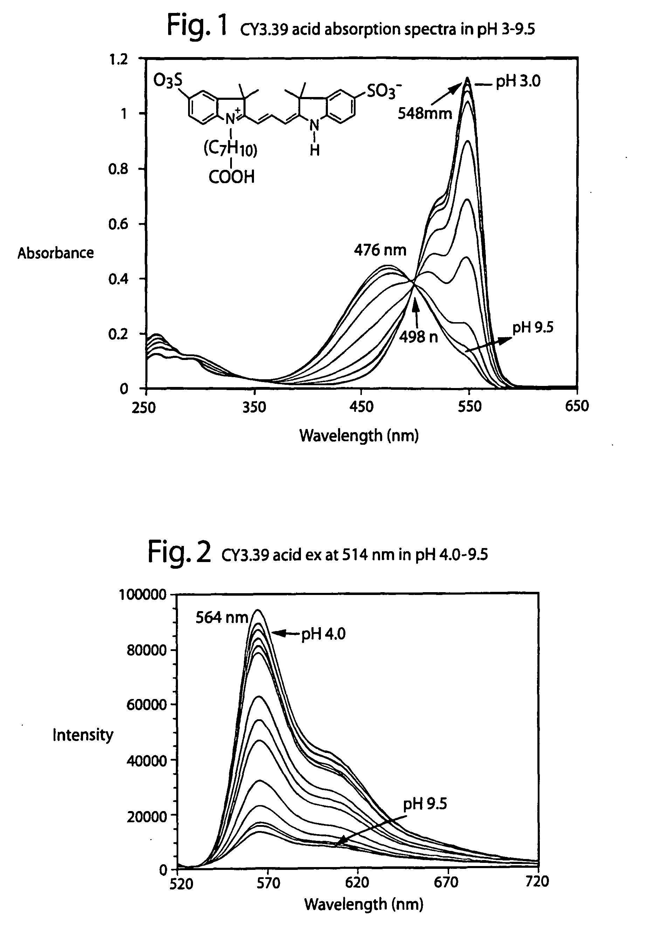

Figure 1 shows the absorbance spectra at pH 3 to pH 9.5 of an exemplified compound, Cy3.39, according to the invention.
Figure 2 shows the emission spectra of an exemplified compound, Cy3.39, according to the invention over a range of pH from pH 4.0 to pH 9.5 with an excitation wavelength of 514 nm.

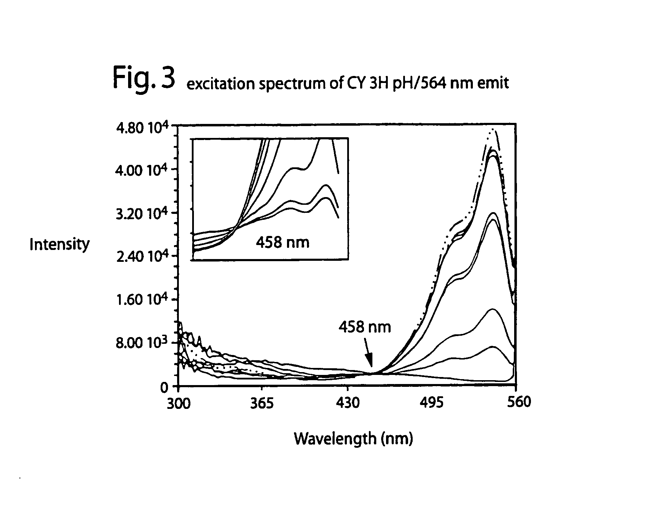

Figure 3 shows the excitation spectra of an exemplified compound, Cy3.39, over a range of pH from 4.0 to pH 9.5 measured at 564 nm emission.

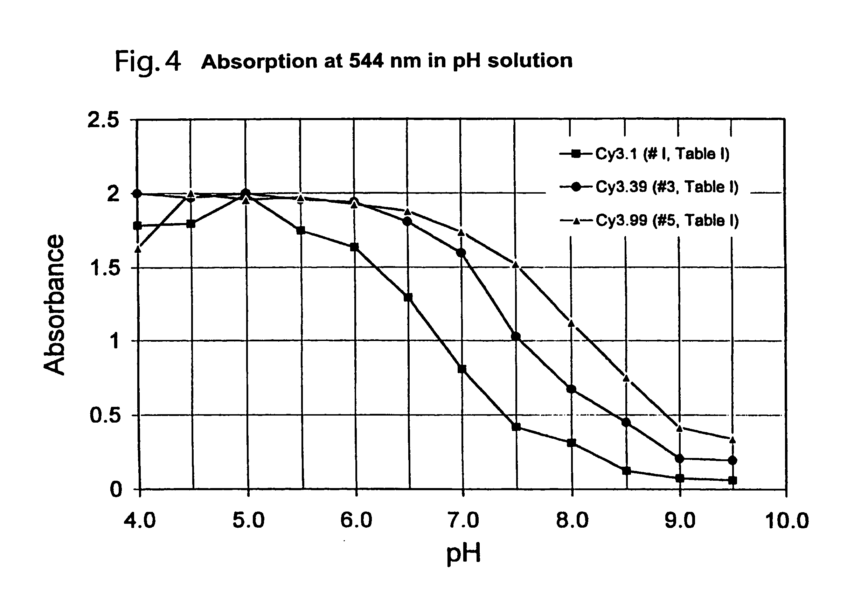

Figure 4 shows the absorbance at pH 4 to 9 of exemplified compounds Cy3.3, Cy3.99 and Cy3.39.
Figure 5 shows the pKa of compound 7.
Figure 6 provides the RFU vs. wavelength for the pH sensitive tandem dye energy transfer cassette-Compound 11 with an excitation of 530 nm.
Figure 7 provides the details of Figure 6 at an excitation of 630 nm.

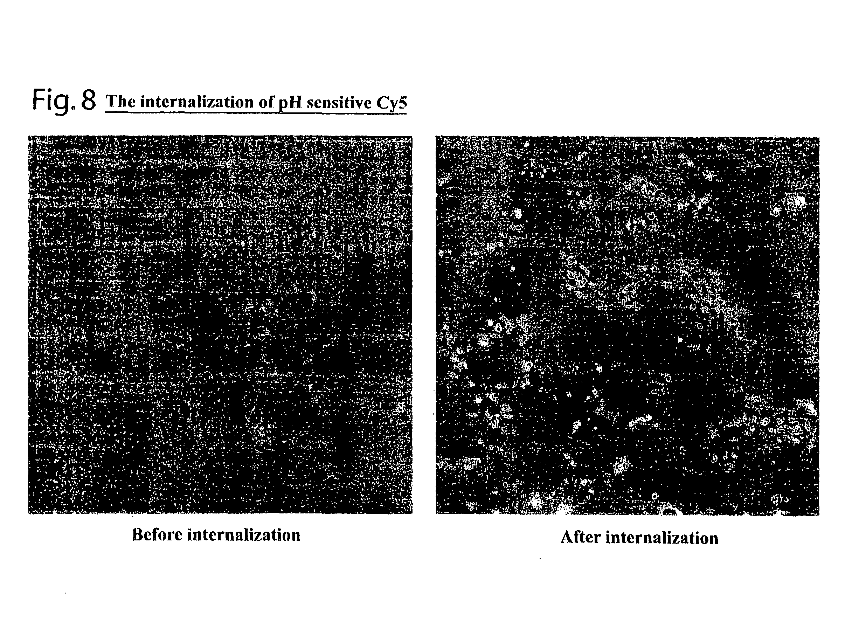

Figure 8 provides an example of the internalization of pH sensitive Cy5, as described herein.

It is an object of the present invention to provide a cyanine derivative which is an acid pH indicator that is water soluble, and fluoresces in the 400-800 nm region of the spectrum.

It is another object of the present invention to provide a fluorescent method of detecting hydrogen ion concentration or pH in the range of about pH 3 to pH 10.

It is another object of the present invention to provide fluorescent reagents which may be used to detect hydrogen ion concentrations or pH in the range of about pH 3 to 10, preferably in the range of about pH 3-6.

In one embodiment, the present invention provides a cyanine derivative of the following formula (I) 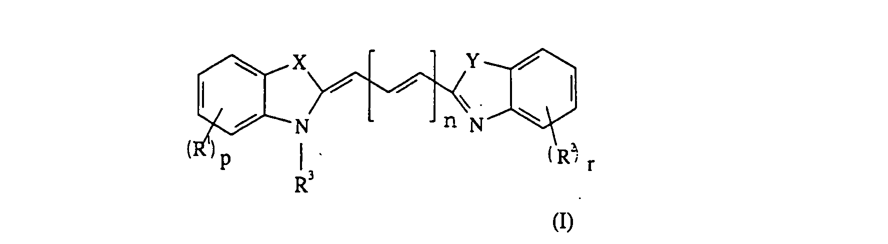 wherein X and Y are selected from >C(C₁-C₄ alkyl)₂, sulfur and oxygen; R¹ and R² are independently selected from H, CH₂NH₂, phosphate, phosphonate, quaternary ammonium, NO₂, (CH₂)_{q}COOH, SO₃⁻, CH₂COOH, NCS, CH₂NH-COR⁷ where R⁷ is C₁-C₂₀ straight or branched alkyl or -(CH₂)_{q}-COOH where q is 0, 1, 2, 3, 4, 5, 6, 7, 8, 9, or 10; R³ is H or is -L-P where L is selected from C₁-C₂₀ straight or branched alkyl optionally containing 0, 1 or 2 unsaturated groups selected from alkenyl, alkynyl and aryl, and P is selected from a reactive group, H, C₁-C₂₀ straight or branched alkyl, SO₃⁻, NH₂, quaternary ammonium, CH₂NH-COR⁸ where R⁸ is C₁-C₂₀ straight or branched alkyl , -(CH₂)ₘ-COOH, NHR⁹ where R⁹ is C₁ to C₂₀ straight or branched alkyl or COOH; n is 0,1, 2 or 3; p and r are independently 0, 1, 2, 3 or 4 and when p and/or r is greater than 1, each R¹ and each R² may be different; and m is 1, 2, 3, 4, 5, 6, 7, 8, 9, or 10, and salts and protonated forms thereof. Each of R¹ and R² may be different and multiply substituted around each of their respective rings such that each ring may contain multiple R¹ and R² substituents and each of the substituents may be different. Each alkyl of X and Y may be different.

Moreover, either or both of R¹, R² or R³ may be joined through a linker or spacer group, L, selected from a straight or branched C₁₋₂₀ alkyl chain, a C₂₋₂₀ monoether or polyether and a C₂₋₂₀ atom containing up to four secondary amide linkages to a fluorescent partner in an energy transfer relationship. L may be attached to a target bonding group or reactive group or reactive moiety, P, as described herein, such as succinimidyl ester, isothiocyanate, thiocyanate, monochlorotriazine, dichlorotriazine, anhydride, haloacetamide, maleimide, sulphonyl halide, phosphoramidite, acid halide, alkylimidate, hydrazide and carbodiimide; and groups reactive with amino, hydroxyl, aldehyde, phosphoryl, or sulphydryl groups. L may include up to two unsaturated groups selected from alkenyl, alkynyl and aryl which may be, for example, interspersed in the chain. Alternatively, the target bonding group may be substituted directly on either of the fluorescent molecules, such as R¹-R⁶, as defined herein.

In such an embodiment of the invention, the dye or derivative of the present invention is one dye of an energy transfer complex as described in European Patent Application No.747700, WO 99/39203 and WO 00/13026, the entire contents of each of which are incorporated herein by reference. The complex comprises a first (or donor) fluorochrome, a second (or acceptor) fluorochrome and at least one linker for covalently attaching the donor and acceptor fluorochromes. Preferably, the energy transfer complex according to the invention contains a target bonding group which is capable of forming a covalent bond with a target material. Alternatively, the energy transfer complex may include a functionality to facilitate cell permeability. Examples include acetoxymethyl ester.

Suitably, in this embodiment, the energy transfer complexes may be used in ratiometric measurements involving changes in pH of a system and in which either the donor or the acceptor species may be a pH sensitive cyanine dye described herein. In the case where the pH sensitive cyanine dye is the donor, then the intensity of fluorescence emission of the acceptor dye is measured after excitation of both the donor and the acceptor dye species independently. In the case where the pH sensitive cyanine dye is the acceptor, then the donor species only is subjected to excitation and the intensity of the emission of both the donor and the acceptor dyes may be measured independently, either sequentially or at the same time. The method therefore provides for a ratiometric measurement in which two dyes which are in energy transfer relationship can be used to provide concentration independent information relating to pH changes in biological and other systems.

The present invention provides a substituted cyanine dye which becomes fluorescent upon protonation of the indolic nitrogen under acidic or basic conditions, depending on the basicity of the dye. That is, for example, in acidic conditions, the protonated cyanine of formula (I) has the following structure 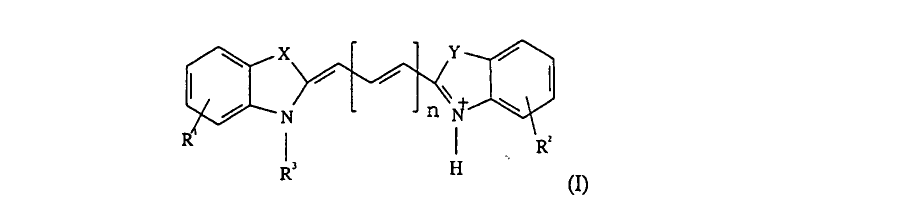 and is fluorescent.

In another embodiment, the present invention provides pH sensitive dyes of the following formula (Ia) 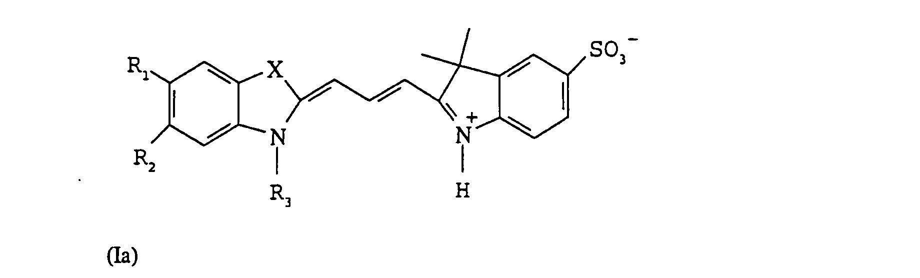 wherein R₁, R₂, and R₃ as are shown below

**Table 1.**

| Compounds of Formula Ia | | | | |
|---|---|---|---|---|
| Type | X | R₁ | R₂ | R₃ |
| 1 | -C(CH₃)₂ | SO₃⁻ | H | H |
| 2 | -C(CH₃)₂ | SO₃⁻ | H | -(CH₂)₁₋₂₀-CH₃ |
| 3 | -C(CH₃)₂ | SO₃⁻ | H | -(CH₂)₁₋₅-COOH |
| 4 | -C(CH₃)₂ | SO₃⁻ | H | -(CH₂)₁₋₅-NHR |
| 5 | -C(CH₃)₂ | H | H | -(CH₂)₁₋₅-NHR |
| 6 | -C(CH₃)₂ | -CH₂-COOH | H | -(CH₂)₁₋₂₀-CH₃ |
| 7 | -C(CH₃)₂ | -CH₂NHR | H | -(CH₂)₁₋₂₀-CH₃ |
| 8 | O | SO₃⁻ | H | -(CH₂)₁₋₂₀-CH₃ |
| 9 | O | SO₃⁻ | H | -(CH₂)₁₋₅-COOH |
| 10 | O | SO₃⁻ | H | -(CH₂)₁₋₅-NHR |
| 11 | O | H | SO₃⁻ | -(CH₂)₁₋₂₀-CH₃ |
| 12 | O | H | SO₃⁻ | -(CH₂)₁₋₅-COOH |
| 13 | O | H | SO₃⁻ | -(CH₂)₁₋₅-NHR |
| 14 | S | SO₃⁻ | H | -(CH₂)₁₋₂₀-CH₃ |
| 15 | S | SO₃⁻ | H | -(CH₂)₁₋₅-COOH |
| 16 | S | SO₃⁻ | H | -(CH₂)₁₋₅-NHR |
| 17 | O | H | CH₂COOH | -(CH₂)₁₋₂₀-CH₃ |
| 18 | O | H | H | -(CH₂)₁₋₅-COOH |
| Unprotonated forms of formula (Ia) are those wherein hydrogen on the quaternary nitrogen is removed and are inclusive herein. R is C₁ to C₂₀ straight or branched alkyl | | | | |

In yet another embodiment, the invention provides derivatives of formulas (I) and (Ia) which contain a substituent to make them covalently reactive with amine, hydroxyl, aldehyde and sulfhydryl groups. These substituents are useful for covalently attaching the cyanine dyes of formulas (I) and (Ia) to biological materials, non-biological molecules and macromolecules and particles so that these materials may be fluorescently labeled; detected and/or quantified by fluorescent detection methods.

In this embodiment, the derivatives of formulas (I) and (Ia) include compounds of the following formulas (II), (III) and (IV), including the protonated forms thereof, 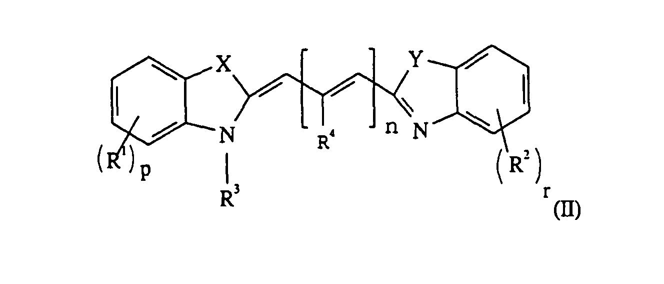 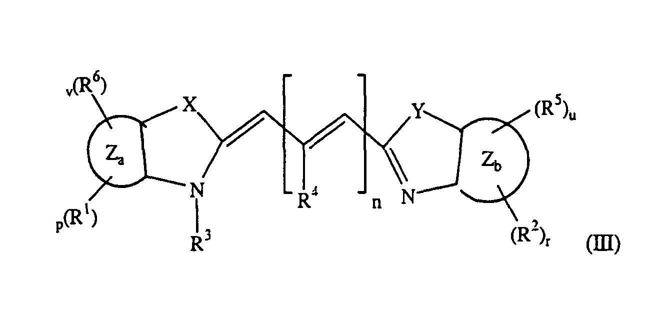 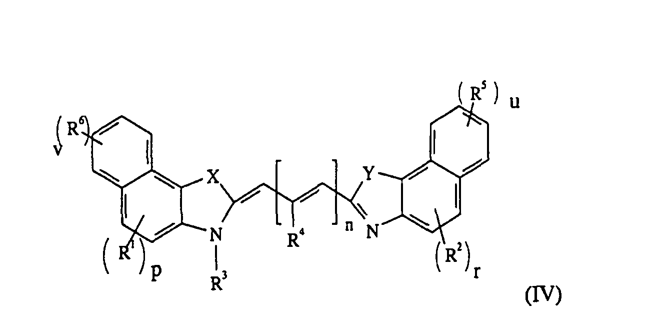 wherein X and Y are selected from >C(C₁-C₄alkyl)₂, sulfur, and oxygen; Zₐ and Z_{b} each independently represent the atoms necessary to complete 1 or 2 fused aromatic rings having 6 carbon atoms containing 0, 1 or 2 nitrogen atoms; R¹, R², R⁵ and R⁶ are independently selected from H, CH₂NH₂, SO₃⁻, phosphate, phosphonate, quaternary ammonium, NO₂, (CH₂)_{q}P, (CH₂)_{q}COOH, CH₂NH-COR⁷ where R⁷ is C₁-C₂₀ straight or branched alkyl or -(CH₂)ₘ-COOH where q is an integer from 0-10; R³ is H or -L-P; where L is selected from C₁-C₂₀ straight or branched alkyl optionally containing 0, 1 or 2 unsaturated groups selected from alkenyl, alkynyl and aryl, and P is selected from a reactive group, H, SO₃⁻, NH₂, quaternary ammonium, CH₂NH-COR⁸ where R⁸ is C₁-C₂₀ straight or branched alkyl , -(CH₂)ₘ-COOH, NHR⁹ where R⁹ is C₁ to C₂₀ straight or branched alkyl or COOH; R⁴ is H or (CH₂)ₘP; n is an integer from 0-3; p, r, u and v are independently 0, 1, 2, 3 or 4 and where p, r, u and/or v is greater than 1, each R¹, R², R⁵ and R⁶ may be different, and m is an integer from 1-10, and salts and protonated derivatives thereof. As described herein, the reactive groups may attach the dye to a component to be labeled.

Any of R¹, R² and R⁴ may independently include a spacer or linker, L, as described above, and/or may include a target bonding group or reacting group or reactive moiety, as described herein, such as succinimidyl ester, isothiocyanate, anhydride, haloacetamide, maleimide, sulphonyl halide, phosphoramidite, acid halide, alkylimidate, hydrazide, and carbodiimide; and groups reactive with amino hydroxyl, aldehyde, phosphoryl, or sulphydryl groups. For certain reagents, at least one of P (where R³ is -(CH₂)ₘ-P), R¹, R², and R⁴ groups on each molecule may also be a group that increases the solubility of the chromophore or affects the selectivity of labeling of the labeled component or affects the position of labeling of the labeled component by the dye. More than one of each of R¹, R², R⁵ and R⁶ may be contained on any ring and each substituent may be the same or different.

In a preferred embodiment of the present invention, at least one of R¹, R² or P (where R³ is -(CH₂)ₘ-P) contains a sulfonate.

In one embodiment, at least one of R⁵ and R⁶, if present, is at least one sulfonate group; where sulfonate includes sulfonic acid as the sulfonate group is merely an ionized sulfonic acid. R⁵ or R⁶ may be reactive moieties, such as primary amines, for example, NHR¹⁰, where R¹⁰ is C₁-C₂₀ straight or branched alkyl. Reactive groups that may be attached directly or indirectly to the chromophore to form any of P (where R³ is -(CH₂)ₘ-P), R¹, R², and R⁴ groups which may include reactive moieties such as, for example, groups containing isothiocyanate, isocyanate, monochlorotriazine, dichlorotriazine, mono- or di-halogen, substituted pyridine, mono- or di-halogen substituted diazine, maleimide, aziridine, sulfonyl halide, acid halide, hydroxy succinimide ester, hydroxy sulfosuccinimide ester, imido ester, alkylimidate, hydrazide carbodiimide hydrazine, azidonitrophenyl, azide, 3-(2-pyridyl dithio)- proprionamide, glyoxal and aldehyde.

The target bonding group, reactive group or reactive moiety, such as may be any of R¹, R² and R⁴, as described herein, may be a group reactive with an amine, hydroxy and/or sulfhydryl groups which are, for example, covalently attached to proteins, nucleic acids, carbohydrates, sugars, cells and combinations thereof, and other biological and non-biological materials, to make these materials fluorescent and detectable, as described in U.S. Patent No. 6,048,982. The dyes of the present invention may be used therefore, to label, for example, avidin, antibodies, DNA, RNA or lectins to detect, measure and/or quantify, for example, biotinylated materials, antigens, haptens, carbohydrate groups, DNA, RNA and complimentary DNA or RNA, such as described therein. Specific examples of P, R¹, R², and R⁴ groups that are especially useful for labeling components with available amino, hydroxyl and sulfhydryl group include:

-NCS;

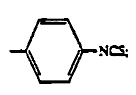

-(CH₂)ₛNCS ; -NCO;

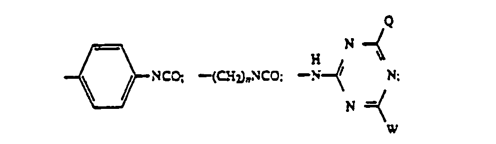

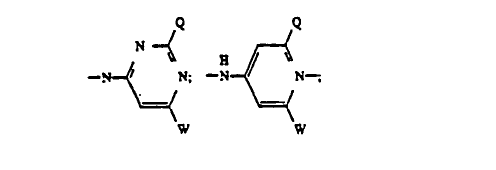

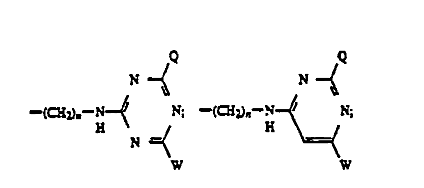

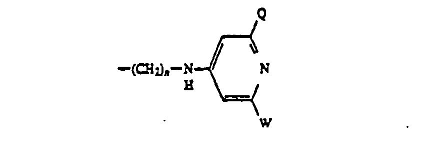

where at least one of Q or W is a leaving group such as I, Br, Cl,

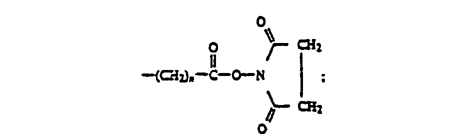

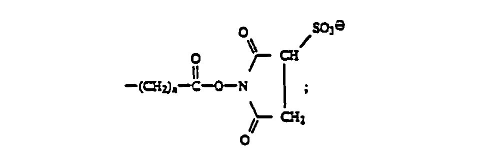

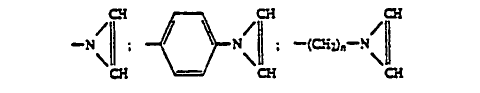

Specific examples of P (where R³ is -(CH₂)ₘ-P), R¹, R², and R⁴ groups that are especially useful for labeling components with available sulfhydryls which can be used for labeling antibodies in a two step process include: 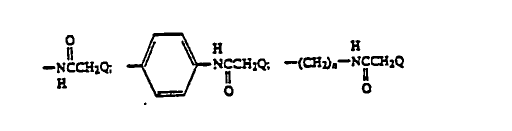 where Q is a leaving group such as I or Br, 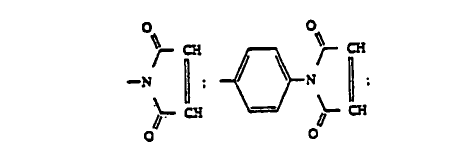 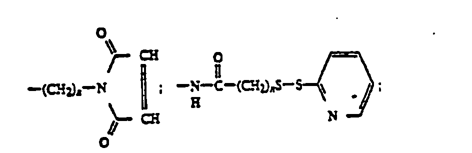 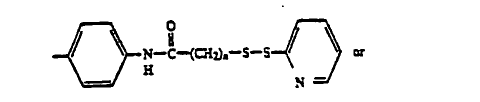 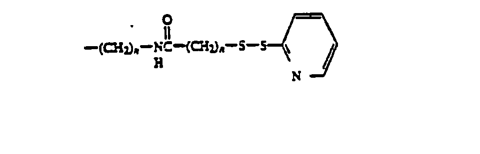 where n is 0 or an integer from 1-8.

Specific examples of P (where R³ is -(CH₂)ₘ-P), R¹, R², and R⁴ groups that are especially useful for labeling components by light-activated cross linking include

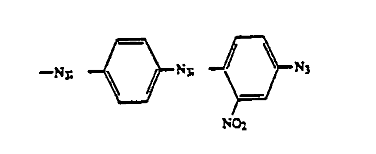

For the purpose of increasing water solubility or reducing unwanted nonspecific binding of the labeled component to inappropriate components in the sample or to reduce the interactions between two of more reactive chromophores on the labeled component which might lead to quenching of fluorescence, the P (where R³ is -(CH₂)ₘ-P), R¹, R², and R⁴ groups can be selected from well known and electrically charged chemical groups. Examples are E-F where F is hydroxyl, phosphate, phosphonate, sulfonate, sulfate, carboxylate, substituted amino or quaternary amino and where E is a spacer group such as -(CH₂)ₜ- where t is 0, 1, 2, 3, 4, or 5. Useful examples include lower alkyl sulfonate, for example, ―(CH₂)₄-SO₃⁻.

In another embodiment, the present invention provides pH sensitive dyes of the following formulas (IIIa) and (IIIb) 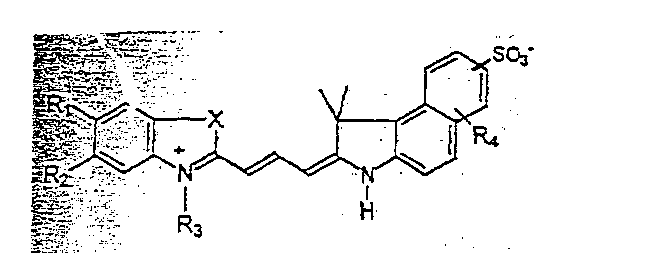 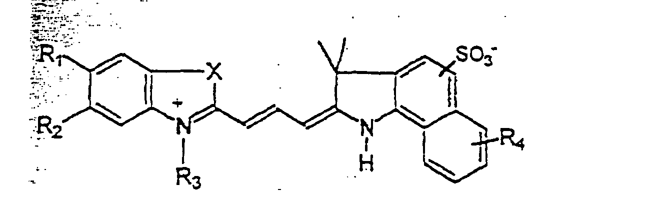 wherein X, R₁, R₂, R₃, and R₄ are as shown below (Table 2)

**Table 2.**

| Compounds of Formulas (IIIa) and (IIIb) | | | | | |
|---|---|---|---|---|---|
| Type | X | R, | R₂ | R₃ | R₄ |
| 1 | -C(CH₃)₂ | SO₃⁻ | H | H | SO₃⁻ or H |
| 2 | -C(CH₃)₂ | SO₃⁻ | H | -(CH₂)₁₋₂₀-CH₃ | SO₃⁻ or H |
| 3 | -C(CH₃)₂ | SO₃⁻ | H | -(CH₂)₁₋₅-COOH | SO₃⁻ or H |
| 4 | -C(CH₃)₂ | SO₃⁻ | H | -(CH₂)₁₋₅-NHR | SO₃⁻ or H |
| 5 | -C(CH₃)₂ | -CH₂-COOH | H | -(CH₂)₁₋₂₀-CH₃ | SO₃⁻ or H |
| 6 | -C(CH₃)₂ | -CH₂-NHR | H | -(CH₂)₁₋₂₀-CH₃ | SO₃⁻ or H |
| 7 | O | SO₃⁻ | H | -(CH₂)₁₋₂₀-CH₃ | SO₃⁻ or H |
| 8 | O | SO₃⁻ | H | -(CH₂)₁₋₅-COOH | SO₃⁻ or H |
| 9 | O | SO₃⁻ | H | -(CH₂)₁₋₅-NHR | SO₃⁻ or H |
| 10 | O | H | SO₃⁻ | -(CH₂)₁₋₂₀-CH₃ | SO₃⁻ or H |
| 11 | O | H | SO₃⁻ | -(CH₂)₁₋₅-COOH | SO₃⁻ or H |
| 12 | O | H | SO₃⁻ | -(CH₂)₁₋₅-NHR | SO₃⁻ or H |
| 13 | S | SO₃⁻ | H | -(CH₂)₁₋₂₀-CH₃ | SO₃⁻ or H |
| 14 | S | SO₃⁻ | H | -(CH₂)₁₋₅-COOH | SO₃⁻ or H |
| 15 | S | SO₃⁻ | H | -(CH₂)₁₋₅-NHR | SO₃⁻ or H |
| 16 | O | H | CH₂COOH | -(CH₂)₁₋₂₀-CH₃ | SO₃⁻ or H |
| 17 | O | H | H | -(CH₂)₁₋₅-COOH | SO₃⁻ or H |
| Unprotonated forms of Formulas (IVa) and (IVb) are those wherein hydrogen on the quaternary nitrogen is removed and are also included in the present invention. R is a straight or branched C₁ to C₂₀ alkyl. | | | | | |

In another embodiment, the present investigation provides pH sensitive dyes of the following formulas (IVa) and (IVb)

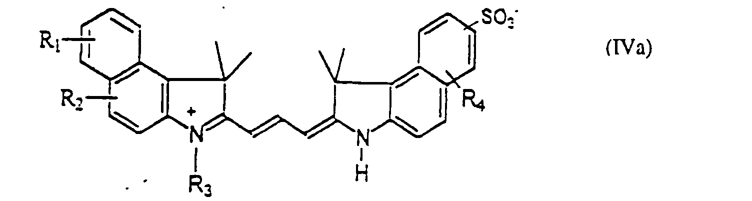

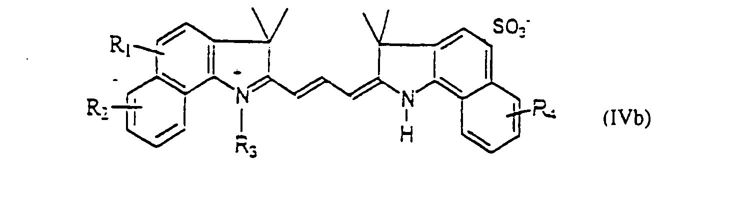

**Table 3.**

| Compounds of Formula (IVa) and (IVb) | | | | |
|---|---|---|---|---|
| | R₁ | R₂ | R₃ | R₄ |
| 1 | SO₃⁻ | SO₃⁻ or H | H | SO₃⁻ or H |
| 2 | SO₃⁻ | SO₃⁻ or H | -(CH₂)₁₋₅-COOH | SO₃⁻ or H |
| 3 | SO₃⁻ | SO₃⁻ or H | -(CH₂)₁₋₅-NHR | SO₃⁻ or H |
| 4 | -CH₂-NHR | H | -(CH₂)₁₋₂₀-CH₃ | SO₃⁻ or H |
| 5 | -CH₂-NHR | H | H | SO₃⁻ or H |
| 6 | SO₃⁻ | SO₃⁻ or H | -(CH₂)₁₋₂₀-CH₃ | SO₃⁻ or H |
| 7 | SO₃⁻ | SO₃⁻ or H | -(CH₂)₁₋₅-COOH | SO₃⁻ or H |
| 8 | SO₃⁻ | SO₃⁻ or H | -(CH₂)₁₋₅-NHR | SO₃⁻ or H |
| 9 | H | SO₃⁻ | -(CH₂)₁₋₅-COOH | SO₃⁻ or H |
| 10 | H | SO₃⁻ | -(CH₂)₁₋₅-NHR | SO₃⁻ or H |
| 11 | SO₃⁻ | H | -(CH₂)₁₋₅-COOH | SO₃⁻ or H |
| 12 | SO₃⁻ | H | -(CH₂)₁₋₅-NHR | SO₃⁻ or H |
| 13 | H | H | -(CH₂)₁₋₅-COOH | SO₃⁻ or H |
| Unprotonated forms of Formulas are those wherein hydrogen on quaternary nitrogen is removed and are included as embodiments of the present invention. R is straight or branched C₁-C₂₀ alkyl. | | | | |

In another embodiment, the invention provides acetoxymethyl ester and acetate esters derivatives of formulas (I), (Ia), (II), (III), (IIIa), (IIIb), (IV), (IVa) and (IVb). Such esters are permeant to membranes, such as biological membranes, and are hydrolyzed by esterases, such as are found in cells. The compounds of the present invention may not require derivatization or modification to be cell permeable. One of ordinary skill will appreciate the variability of the cell permeability of the compounds of the present invention and be able to routinely test for the same.

The polymethine chain of the dyes of the present invention may also contain one or more cyclic chemical groups that form bridges between two or more of the carbon atoms of the polymethine chain. These bridges might serve to increase the chemical or photostability of the dye and might be used to alter the absorption and emission wavelength of the dye or change its extinction coefficient or quantum yield. Improved solubility properties in aqueous environments may be obtained by this modification.

In another embodiment, the present invention provides a component-labeled complex wherein the label is a compound of formulas (I), (II), (III) and (IV) and the component is an antibody, protein, peptide, enzyme substrate, hormone, lymphokine, metabolite, receptor, antigen, hapten, lectin, avidin, streptavidin, toxin, carbohydrate, oligosaccharide, polysaccharide, nucleic acid, derivatized deoxy nucleic acid, DNA fragment, RNA fragment, derivatized DNA fragment, derivatized RNA fragment, natural drug, synthetic drug, virus particle, bacterial particle, virus component, yeast component, blood cell, blood cell component, plasma component, serum component, biological cell, noncellular blood component, bacteria, bacterial component, natural or synthetic lipid vesicle, poison, environmental pollutant, polymer, polymer particle, glass particle, glass surface, plastic particle, plastic surface, polymer membrane, conductor or semiconductor.

A cyanine dye or its derivative of the present invention preferably absorbs maximally at a pH of 3-10, more preferably, at a pH of 3-6, in the range of 400-800 nm, alternatively, in the ranges of 520-580 nm, or 650-670 nm, preferably in the range of 530-550 nm, more preferably in the range of 540-550 nm, most preferably about 548 nm with an isosbestic point in the range of 495-500 nm, preferably about 498 nm.

A cyanine dye or its derivative of the present invention fluoresces maximally in acidic conditions (pH of 4-7, preferably a pH of 4-5, most preferably a pH of 4), however, some compounds of the present invention fluoresce under basic conditions, such as at a pH in the range of 7.2-10, at about 560-580 nm, preferably 560-565, most preferably at 564 nm; when excited by a wavelength of 505-520 nm, preferably 510-515; most preferably 514 nm. One of ordinary skill will appreciate that as n increases in the dyes of the present invention, the wavelength of fluorescence emission will increase and likewise when n decreases, the fluorescent wavelength will decrease.

Some dyes or derivatives of the present invention may preferably be maximally excited with 514 nm spectral line of an argon-ion laser. Dyes may be designed, however, within the scope of the present invention which may be excited and emit fluorescence over a range of wavelengths. The extinction coefficient of dyes or derivatives of the present invention is preferably greater than about 130,000 cm⁻¹ M⁻¹. The fluorescence of the dyes and derivatives of the present invention are preferably sensitive to pH between pH 4 and pH 10 and are water soluble.

In another embodiment, the present invention provides a fluorescent method of qualitative and/or quantitative detection of pH. Such methods include contacting or mixing at least one dye of the present invention with a composition of cells, tissues or biological fluid wherein the presence of fluoresence indicates an acidic or, possibly basic, environment. In one embodiment, the present invention present invention provides a method of detecting intracellular acid environments, such as may be contained in subcellular compartments, or organells or structures. The compounds or derivatives of the present invention may be actively or passively adsorbed or absorbed into cells, where they may be detected by fluorescence detection.

In yet another embodiment, the present invention provides a method of fluorescent detection wherein the dye or derivative of the present invention is detected in acidic conditions, such as in a differential, or when multiple fluorescent dyes are used, a dye or derivative of the present invention may be used in conjunction with dyes which do not either fluoresce in acidic conditions and/or at the wavelengths of the presently provided dyes or derivatives.

As noted above, the present invention also provides a pH sensitive, concentration independent, ratiometric measurement method wherein at least a pair of fluorescent dyes are linked in an energy-transfer arrangement, preferably through a linker, L, with optional target bonding groups contained thereon, as described herein. At least one of the dyes of such a conjugate is one of the pH sensitive dyes of the present invention. One of ordinary skill will appreciate that such dye conjugates contain, at a minimum, a dye which is an energy donor and a dye which is an energy acceptor. Where the pH sensitive dye of the present invention is the energy donor, the ratiometric measurement method of the present invention involves determining the ratio of fluorescence intensity of the acceptor emission after excitation of each of the donor and acceptor. When the pH sensitive dye of the present invention is the energy acceptor, then the ratio of the maximum fluorescence intensity of both the donor and acceptor is determined from excitation at the maximum absorption of the donor species.

The methods according to the present invention may employ known devices for illumination and detection at separate defined wavelengths, such as first and second wavelengths, respectively. Such devices include, but are not limited to, fluorescence spectrometers, absorption spectrophotometers, fluorescence microscopes, transmission light microscopes, flow cytometers, fiberoptic sensors, immunoassay instruments and video fluorescent imaging devices. Methods of the present invention include those described n the "Handbook of Fluorescent Probes and Research Chemicals" published by Molecular Probes, Inc. And "Fluorescent Probes in Cellular and Molecular Biology" by Slavik (1994) CRC Press, Inc., especially methods where measurement of acidic conditions are preferred, wherein the dye and/or derivative of the present invention is employed.

The method of this invention can also employ chemical analysis methods to detect attachment of the dye or derivative of the present invention to the labeled component or components. Chemical analysis methods can include infrared spectrometry, NMR spectrometry, absorption spectrometry, fluorescence spectrometry, mass spectrometry and chromatographic methods.

The dyes and derivatives of the present invention may be made by methods well known in the art, such as by methods described in U.S. Patent No. 5,486,268, U.S. Patent No. 5,486,616, and U.S. Patent No, 4,981,977, the entire contents of which are incorporated wherein by reference.

The following exemplification of the present invention further describes preferred embodiments.

### Example 1: Synthesis of Cy3.39.OH (Compound 1), intermediates and derivatives thereof

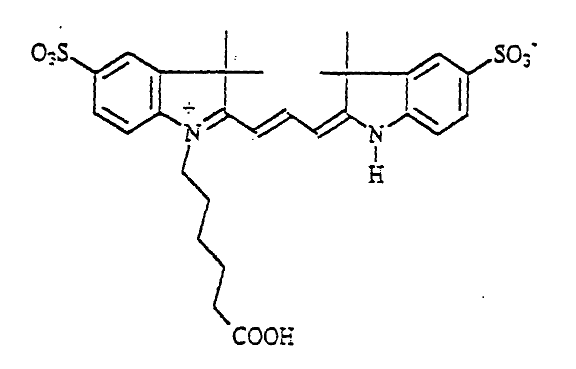

### 1.1 Synthesis of 5-sulfo-2, 3, 3-trimethylindolenine (I)

Conventional Fisher Indole synthesis was used as follows. In a 2-L three necked flask equipped with mechanical stirrer and reflux condenser was added acetic acid (300 mL), 3-methyl-2-butanone (168 mL, 1.59 mol) and p-hydrazinobenzenesulfonic acid (100 g, 0.53 mol). The mixture was heated to reflux for three hours and then cooled for several hours when pink solid separated. (93 g, 75%, mp. 290-95 °C). ¹H NMR (D₂O), δ, 1.4 (s, 6H C-(CH₃)₂); 6.5 (d, 1H, J = 7 Hz, 7-H); 7. 1 (dd, 1H, J = 7.0, 1.2 Hz, 6-H); 7.13 (s, 1H, 4-H). Singlet for 2-methyl is not seen in D₂O, but it appears when nmr is recorded in DMSO d₆.

To a stirred solution of sulfoindolenine in methanol was added a saturated solution of potassium hydroxide in isopropanol. The alkaline solution turns yellow and the potassium salt of the sulfoindolenine precipitated as yellow solid almost quantitatively. ¹H NMR (D₂O), δ, 7.13 (s, 1 H 4-H); 7. 1 (dd, 1 H, J = 7.0, 1.2 Hz, 6-H); 6.5 (d, 1 H, J = 7 Hz, 7-H); 2.2 (s, 3H, C-CH₃); 1.4 (s, 6H, -C(CH₃)₂); Rf = 0.5 (Reverse phase C18, water).

### 1.2 Preparation of Salt free sulfoindolenine

The potassium salt of sulfoindolenine was dissolved in water and passed through a column of Dowex, strongly acidic hydrogen ion exchange resin. Potassium free sulfoindolenine was obtained as brown powder. This is essential to ensure that the sulfoindolenine is an internal salt and the nitrogen is protonated.

### 1.3 5-Sulfo-1-(γ-carboxypentynyl)-2,3,3-trimethylindolenine (II).

The potassium salt of 5-sulfo-2, 3, 3,-trimethylindolemine (I) and 6-bromohexanoic acid (1.2 eq) were mixed in 1,2-dichlorobenzene and heated at 110°C for 12 h. Mixture was cooled, 1,2-dichlorobenzene was decanted and the solid was triturated with isopropanol until free powder is obtained. λmax (water) = 274 mn; ¹H NMR (D₂O), δ, 8.13 (s, 1 H, 4-H); 8.03 (d, 1 H, J = 9 Hz. 1.1Hz 6-H); 7.2 (d, 1 H, J = 9 Hz, 7-H); 4.51 (t 2H, J = 7.5 Hz, α-CH₂-); 2.25 (t, 2H, J = 7.5 Hz, ε-CH₂-); 1.99 (m, 2H, β-CH₂); 1.35-1.66 (m, 4H, γ-CH₂, δ-CH₂); 1.61 (s, 6H,-(CH₃)₂). Protons of the 2-methyl are not seen in D₂O Rf = 0.27 (reversed phase, water).

### 1.4 Preparation of Intermediate 415 (III)

A mixture of quaternary salt of indolenine (II, 5.3 g, 0,015 mol) and N,N'diphenylformamidine (3.3 g, 0.0 17 mol) in acetic acid (20 mL) was heated to reflux. The completion of the reaction was monitored by absorption spectra in methanol which showed that as the reaction progressed the absorption maximum at 286 nm declined almost to the vanishing point and the absorption at 415 nm rose correspondingly (about 3-4 h). Extended heating produced some symmetrical dye (>5%). Acetic acid was removed on a rotary evaporator and the crude orange powder was immediately used for next reaction. λmax (water) = 415 nm; The small portion of the reaction mixture was purified on a reversed phased 18C column using water-methanol mixture as eluent. ¹H NMR (D₂O) δ, 8.15 (s, 1H, 4-H); 8.0 (d, 2H, J = 7.0 Hz, 6-H); 7.9 (d, 1H, J = 7 Hz, 7H); 7.1-7.55 (m, 7H, 2 vinyl and 5 aromatic protons of aniline) 4.53 (t, 2H, J = 7.5 Hz, α-CH₂-); 2.3 (t, 2H, J = 7.5 Hz, ε-CH₂-), 1.99 (m, 2H, β-CH₂); 1.4-1.7 (m, 4H, γ-CH₂, δ-CH₂); 1.61 (s, 6H, - (CH₃)₂). Rf = 0.5 (RP-C18, 25% methanol in water).

### 1.5 Preparation of dye. Cy3.39.OH

The intermediate (III, 5.9 g, 0.013 mol) was redissolved in acetic anhydride (25 mL) and pyridine (25 mL). The mixture was warmed when absorption at 415 nm disappeared almost immediately and an additional band appeared at 385 nm. Indolenine (potassium free) (5.3 g, 0.015 mol) was added and the mixture was heated at 110°C till absorption maxima at 385 nm was almost vanished. It was cooled and the dye precipitated with ethyl ether. After washings with isopropanol (X10 mL) the solid was recovered by filtration and purified on reverse phase C 18 using water:methanol mixture as eluent (2.3 g, yield 28%) ¹H NMR (D₂O) δ, 8.3-8.8 (m, 5H, pyridine protons); 8.2 (t, 1H, J = 14 Hz, β-proton of the bridge); 7.5-7.9 (m, 4H, 4-H, 4'-H, 6-H & 6'-H); 7.1-7.2 (2d, 2H, 7-H & 7'-H); 6.3-6.1(m, 2H, α, α' protons of the bridge); 4.0 (m, 2H, α-CH₂-); 2.4 (t, 2H, J = 7 Hz, -CH₂COOH); 1.4-2.0 (m, 12H, three -CH₂, and -(CH₃)₂).

Two symmetrical dyes Cy3.18.OH (from sulfoindolenine (II)) and Disulfo DiI -(3) (from sulfoindolenine (I)) were also obtained as side products.
TLC RP-C18, (water:methanol-8.5:1.5)
Rf = 0.65, Cy3.18.OH
0.50, Cy3.39.OH
0.44, Disulfo DiI -(3)

### 1.6 Succinimidyl Ester of Cy3.39.OH

In a typical experiment carboxyalkyl sulfocyanine was dissolved in a mixture of dry DMF (2 mL/100 mg dye) and dry pyridine (0.1 mL/100 mg dye). Disuccinimidyl carbonate (DSC) (1.5 eq) was added and the mixture was stirred at 55-60°C for 90 min. under nitrogen. After diluting the mixture with dry ethyl ether, the supemant was decanted. The product was washed repeatedly with ethyl ether and filtered. Nearly quantitative yield of cyanine active ester was obtained.

### 1.7 Reaction of Cy3.39.OSu with antibody

Stock solutions of the Cy3.39 active ester was made in dry DMF (1 mg/100µL). One mg sheep IgG (6.45mM) was dissolved in 250 µL 0.1M carbonate-bicarbonate buffer (pH 9.4) and the desired amount of dye was added during vigorous vortex mixing. The reaction mixture was kept for 15 minutes at room temperature. The unconjugated dye was separated from the protein by gel permeation chromatography over Sephadex G-50 (0.7 X 20cm column) using pH 7 buffer solution as eluent.

### Example 2: Synthesis of Cy3.1 (Compound 2)

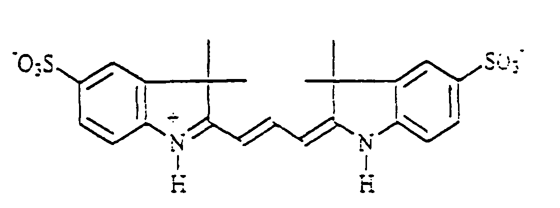

A mixture of 2,3,3,-trimethylindolenine-5-sulfonate (2 mmoL) and ethylorthoformate (1 mmoL) in pyridine (5 mL) was heated to reflux for 10 min. The reaction mixture was cooled and diluted with several volumes of anhydrous ether and chilled in an ice bath. Scratching with a glass rod caused solidification of the product as a red powder, which was collected on a filter and dried (yield 60%). The product was shown to be almost pure by TLC (Rf = 0.44, C18 reversed phase plate, water/methanol, 8:2 v/v). Pyridinium salt of Cy3.1 was dissolved in water and passed through a column of Dowex, strongly acidic hydrogen ion exchange resin. ¹H NMR (D₂O) δ, 8.3 (t, 1H J = 13 Hz, β-proton of the bridge); 7.8 (s, 2H 4-H & 4'-H); 7.6 (d, 2H, J = 7 Hz, 6-H, 6'-H); 7.1-7.2 (2d, 2H J = 7 Hz, 7-H & 7'-H); 5.9 (broad s, 2H, α, α' protons of the bridge); 1.4-(broad s, 12H -(CH₃)₂).

Alternatively, the dye was obtained by heating sulfoindolenine (I) with ethyl orthoformate in acetic acid for 2 hr. Acetic acid was removed on a rotary evaporator under reduced pressure and the residue was triturated with anhydrous ethyl ether to obtained red solid, which was purified by C18 reversed phase flash column chromatography (yield 25%).

### Example 3: Synthesis of Cy3.99.OH (Compound 3) and its succinimidyl ester

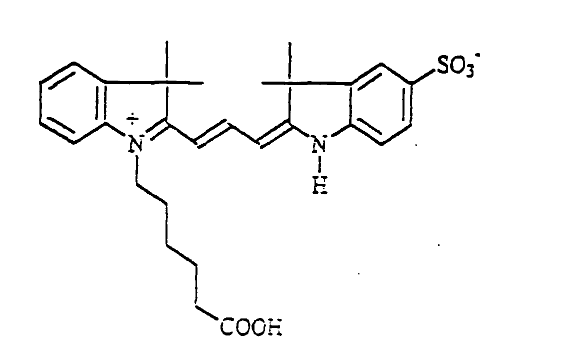

The dye and its succinimidyl ester were prepared according to the procedure described for Cy3.39.OH. The dye is water insoluble. Its water soluble dextran conjugate was prepared as described below.

To a solution of aminodextran (Mr 40,000, approx. 7.2 amino groups per dextran molecule) (8.5 mg, 0.2mmoL) in 0.02 M carbonate buffer was added a solution of active ester (2 mg) in a dry DMF. The mixture was stirred for 30 minutes at room temperature. The dye-dextran conjugate was separated from nonconjugated dye by sephadex G-50 gel permeation chromatography using ammonium acetate (50mM) as elution buffer. An average of 2.2 dye molecules was covalently linked to each dextran molecule.

### Example 4: 5-(Carboxymethyl)-2-[1E,3E)-5-(3,3-dimethyl-5-sulfo-1,3-dihydro-2H-indol-2-ylidene)-1,3-pentadienyl]-3,3-dimethyl-1-(4-sulfobutyl)-3H-indolium (Compound 4)

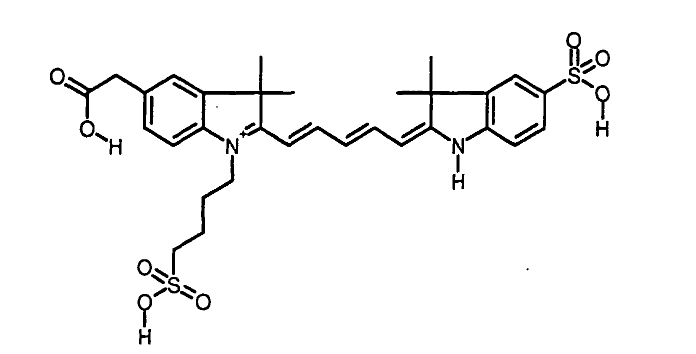

### 4.1 5-Carboxymethyl-2,3,3-trimethyl-1-(4-sulfobutyl)-3H- indolium

5-Carboxymethyl-2,3,3-trimethyl indole (100mg, 0.38mmol) and 1,4-butane sultone (43µl, 0.418mmol) were dissolved in 1ml of butyronitrile and heated at 120°C for 24 hours. A pink solid precipitated out of solution. The solution was cooled and the solid removed by filtration. Washing with fresh butyronitrile and drying yielded the product. (122mg, 91%). ¹H NMR (*d*₆-DMSO) δ 7.95 (d, 1H, J = 8.3), δ 7.7 (s, 1H), δ 7.5 (d, 1H, J = 8.28), δ 4.48 (t, 2H , δ-CH₂), δ 3.73 (s, 1H Ar-CH₂), δ 2.83 (s, 3H, 2-Me), δ 2.50 (t, 2H, α-CH₂), δ 1.93 (m, 2H, χ-CH₂), δ 1.73 (m, 2H, β-CH₂), δ 1.5 (s, 6H, 3-(Me)₂. MALDI-TOF m/z = 357 (100%) M⁺ = 355 for C₁₇H₂₅NO₅S, UV (H₂O) λₘₐₓ = 284nm.

### 4.2 5-(Carboxymethyl)-2-[1E,3E)-5-(3,3-dimethyl-5-sulfo-1,3-dihydro-2H-indol-2-ylidene)-1,3-pentadienyl]-3,3-dimethyl-1-(4-sulfobutyl)-3H-indolium

5-Carboxymethyl-2,3,3-trimethyl-1-(4-sulfobutyl)-3*H*-indolium (100mg, 0.27mmol), 5-sulfo-2,3,3-trimethylindolenine (69.3mg, 0.27mmol) and malonaldehyde bis(phenylimine) monohydrochloride (70mg, 0.27mmol) were dissolved in N,N-dimethyl formamide. To this solution was added benzoic acid (66mg 0.54mmol) and benzoic anhydride (122mg, 0.54mmol). The solution was heated at 90°C for 3 hours to yield a dark blue solution. The reaction was monitored by UV until the absorbance of the anil intermediate (454nm) was no longer observed. The solution was cooled and the solvent removed *in vacuo.* The dye was purified by reverse phase HPLC using a Rainin Dynamax 60Å C18 column at 10ml/min with a solvent gradient of 15% B for 5 minutes ramping from 15% to 50% B over 75 minutes, where A = H₂O (0.1% Acetic acid) and B = acetonitrile (0.1% acetic acid). The column retention time was 33 minutes (UV at 650nm). MALDI-TOF m/z = 629 (100%), M⁺ = 630 for C₃₁H₃₈N₂O₈S₂. UV (H₂O/H⁺) λₘₐₓ 650nm, UV (H₂O/OH⁻) λₘₐₓ 515nm.

### 4.3 Compound 4 (N-Hydroxysuccinimidyl ester)

The free acid (1mg, 1.5µmol) was dissolved in anhydrous DMSO (100µL). To this solution was added benzotriazole-1-yl-oxy-tris-pyrrolidino-phosphonium hexafluorophosphate (PyBOP) (0.78mg, 1.5µmol), N-hydroxysuccinimide (0.2mg, 1.5µmol) and diisopropylethylamine (0.25µl, 1.5µmol). The solution reddened. The solution was stirred for 1 hour at room temperature. The reaction mixture was purified by HPLC using the same conditions as for the free acid. The column retention time for the succinimidyl ester was 45 minutes (UV 650nm). MALDI-TOF m/z = 725 (100%), M⁺ = 727 for C₃₅H₄₁N₃O₁₀S₂.

### Example 5: 2-{1E,3E)-5[5-(Carboxymethyl)-3,3-dimethyl-1,3-dihydro-2H-indol-2-ylidene]-1,3-pentadienyl}-1-ethyl-3,3-dimethyl-5-sulfo-3H-indolium (Compound 5)

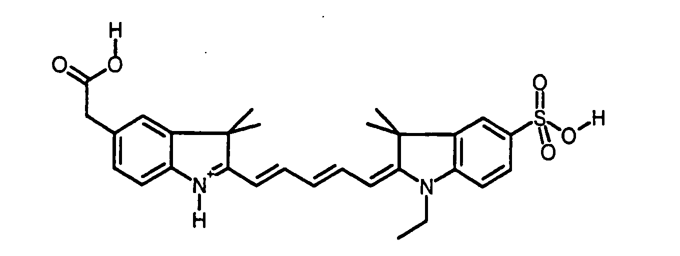

### 5.1 1-Ethyl-2,3,3,-trimethyl-3H-indolium-5-sulfonate

5-Sulfo-2,3,3-trimethylindolenine (potassium salt) (11g, 0.04mol) was suspended in ethyl iodide (40ml) and the reaction refluxed for 24 hours. The mixture was cooled and the excess ethyl iodide was decanted. The light purple solid was triturated with acetone (3 x 50ml) to remove potassium iodide. The powder was dried. ¹H NMR (D₂O) δ 8.13 (s, 1H, 4-H), δ 8.03 (dd, 1H, J = 9,6-H), δ 7.2 (d, 1H, J = 9, 7-H), δ 4.51 (q, 2H, J = 7.5, α-CH₂), δ 1.59 (t, 3H, β-CH₃), δ 1.61 (s, 6H, (CH₃)₂). MALDI-TOF m/z = 268 (100%), M⁺ = 271 for C₁₃H₂₁NO₃S.

### 5.2 2-{1E,3E)-5[5-(Carboxymethyl)-3,3-dimethyl-1,3-dihydro-2H-indol-2-ylidene]-1,3-pentadienyl}1-ethyl-3,3-dimethyl-5-sulfo-3H-indolium

1-Ethyl-2,3,3,-trimethyl-3*H*-indolium-5-sulfonate (20mg; 0.0745mmol), malonaldehyde bis(phenylimine) (21.22mg; 0.082mmol) and acetic anhydride (1ml) at 120°C for 24hrs. The solvent was removed under reduced pressure to give the anil as a red solid. 5-Carboxymethyl-2,3,3-trimethyl indole (16.2mg; 0.0745mmol) was then added and the crude mixture dissolved in a pyridine, acetic acid, acetic anhydride (4.5:4.5:0.5 ratio) solution. The solution was then stirred and heated at 70°C overnight. This was then cooled and the solvent removed *in vacuo* to yield a blue solid which was purified by reverse phase HPLC to yield complex 5 (60%) MALDI-TOF m/z = 518 (100), M⁺ = 520 for C₂₉H₃₂N₂O₅S .UV (H₂O/H⁺) λₘₐₓ 650nm.

### 5.3 Compound 5 (N-Hydroxysuccimidyl ester)

The free acid (1mg, 2.2µmol) was dissolved in DMF (100µl). To this solution was added diisopropylethylamine, (1µl), and *O*-(N-succinimidyl)-N,N,N',N'-bis(tetramethylene)-uronium hexafluorophosphate (1.4eq). The solution was stirred for 30 minutes to yield (10mg). MALDI-TOF m/z = 618 (100%), M⁺ = 617 for C₃₃H₃₅N₃O₇S.

### Example 6: 2-[(E)-5-(Carboxymethyl)-3,3-dimethyl-1,3-dihydro-2H-indol-2-ylidene]-1-pronenyl}-3-(4-sulfobutyl)-1,3-benzothizol-3-ium (Compound 6)

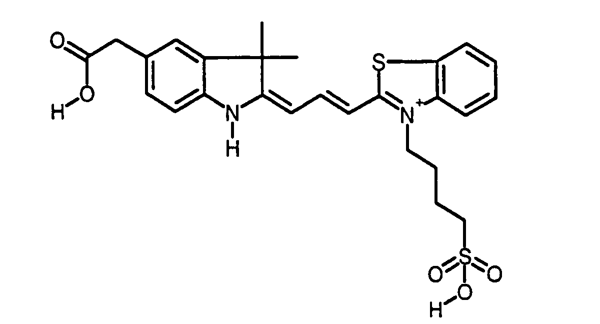

### 6.1 2-Methyl-3-(4-sulfobutyl)-1,3-benzothiazol-3-ium

2-Methyl-benzothiazole (100µl, 0.57mmol) and 1,4-butane sultone (59µl, 0.57mmol) were dissolved in butyronitrile (2ml). The mixture was stirred at 120°C for 24 hours to form a pink precipitate. The solid was filtered and washed with fresh butyronitrile and diethyl ether. ¹H-NMR (*d*_{*6*}-DMSO), δ 8.86 (d, 1H,) δ 8.56 (dd, 1H), δ 8.17 (dd 1H), δ 7.89 (dd, 1H), δ 3.18 (s, 3H, δ 2.96 (t, 2H), δ 2.18 (t, 2H), δ 1.80-1.77 (m, 4H). MALDI-TOF m/z = 285 (100%) M⁺ = 271 for C₁₂H₁₇NO₃S₂.

### 6.2 2-[(E)-5-(Carboxymethyl)-3,3-dimethyl-1,3-dihydro-2H-indol-2-ylidene]-1-propenyl}-3-(4-sulfobutyl)-1,3-benzothizol-3-ium

2-Methyl-3-(4-sulfobutyl)-1,3-benzothiazol-3-ium (10mg, 35µmol), 5-carboxymethyl-2,3,3-trimethyl indole (8mg; 35µmol) and *N,N*'-diphenyl formamidine (7.5mg, 38.5µmol) were stirred in acetonitrile at 70°C. To this was added benzoic acid (8mg, 70µmol) and benzoic anhydride (15mg, 70µmol). The solution was heated for 4 hours and then cooled. The acetonitrile was removed *in vacuo* to yield a dark pink gum. This was purified by reverse phase HPLC using a Rainin Dynamax 60Å C18 column at 10ml/min with a solvent gradient of 15% B for 5 minutes ramping from 15% to 50% B over 75minutes, where A = H₂O (0.1% Acetic acid) andB = acetonitrile (0.1% acetic acid). MALDI-TOF M⁺ = 511 (100%).

### Example 7: 3-(5-Carboxypentyl)-2-[1E,3E)-5-(3,3-dimethyl-5-sulfo-1,3-dihydro-2H-indol-2-ylidene)-1,3-pentadienyl]-1,3-benzothiazol-3-ium (Compound 7)

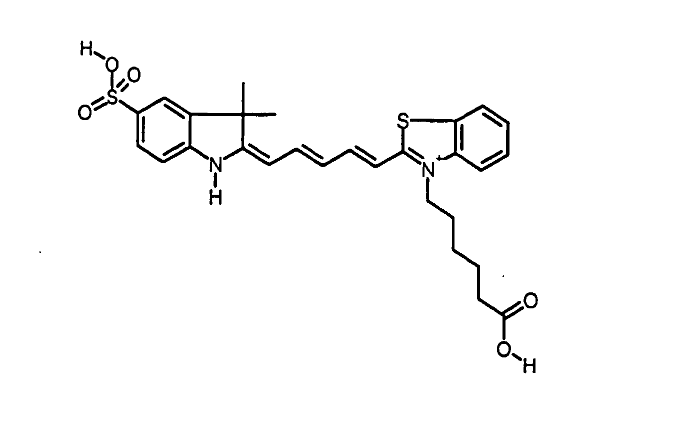

### 7.1 3-(5-carboxypentyl)-2-methyl-1,3-benzothiazol-ium bromide

2-Methylbenzothiazole (100µl, 0.57mmol) was dissolved in toluene (1ml) with 6-bromohexanoic acid (123mg, 0.63mmol). The solution was refluxed for 24 hours. The solution was then cooled to yield a pale solid. This was filtered and washed with cold toluene and then diethyl ether. Yield 58%. ¹H NMR (*d*_{*6*}-DMSO) δ 8.49 (dd, 1H, J = 7.82), δ 8.36 (dd, 1H, J = 7.81), δ 7.92, (dd, 1H, J = 7.32), δ 7.78 (d, 1H, 7.32), δ 4.73 (t, 2H, α-CH₂, J = 15.63), δ 3.24 (s, 3H β-CH₃), δ 2.23 (T, 2H, ε-CH₂, J = 13.6), δ 1.86 (m, 2H, β-CH₂), δ 1.56 - 1.45 (m, 4H, γ- and δ-CH₂). MALDI-TOF m/z = 265 (100%), M⁺ = 265 for C₁₄H₁₉NO₂S.

### 7.2 3-(5-carboxypentyl)-2-[1E,3E)-5-(3,3-dimethyl-5-sulfo-1.3-dihydro-2H-indol-2-ylidene)-1,3-pentadienyl]-1,3-benzothiazol-3-ium

5-Sulfo-2,3,3-trimethylindolenine (120mg, 0.46mmol) was stirred in a 1:1:0.2 mixture of pyridine:acetic acid and acetic anhydride at 120°C. Malonaldehyde bis(phenylimine) mono hydrochloride (120mg, 0.46mmol) was added slowly and the reaction stirred for 30 minutes. 1-(5-carboxypentyl)-2-methyl-benzothiazol-3-ium bromide (122mg, 0.46mmol) was dissolved in a 1:1:0.2 mixture of pyridine, acetic acid and acetic anhydride (1ml) and this was added to the reaction mixture in 100µl portions every 30 minutes. The reaction was heated overnight and then cooled to give a dark blue solution. TLC (20% methanol/CH₂Cl₂) showed the presence of a pH sensitive Cy5. The solution was purified by reverse phase HPLC using a Rainin Dynamax 60Å C18 column at 10ml/min with a solvent gradient of 15% B for 5 minutes ramping from 15% to 50% B over 75minutes, where A = H₂O (0.1% Acetic acid) and B = acetonitrile (0.1% acetic acid). The column retention time was 50.6 minutes. MALDI-TOF m/z = 537 (100%), M⁺ = 540 for C₂₈H₃₂N₂O₅S₂.

### Example 8: 5-(Aminomethyl)-2-[1E,3E)-5-(3,3-dimethyl-5-sulfo-1,3-dihydro-2H-indol-2-ylidene)-1,3-pentadienyl]-1,3,3-trimethyl-3H-indolium (Compound 8)

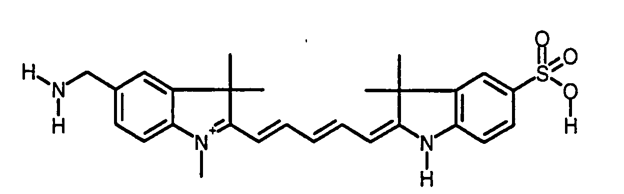

### 8.1 5-[1,3-Dioxo-1,3-dihydro-2H-isoindol-2-yl)methyl]-1,2,3,3-tetramethyl-3H-indolium

2-Methylene-1,3,3-trimethylindolenine (100g, 0.575mol) was slowly added to concentrated sulphuric acid (500ml) at 0°C. This was stirred for 1 hour and N-(hydroxymethyl)-phthalimide (100g, 0.565mol) was added in 10g portions over two hours at 0°C. The reaction was then stirred for 70 hours to give an orange solution. This was poured onto 500g of ice. Concentrated ammonia solution (750ml) was diluted with distilled water (750ml) and slowly added to the quenched reaction mixture at 0°C. Upon neutralisation the aqueous solution became yellow. This was extracted with chloroform and the organic layer dried with sodium sulfate, filtered and the solvent removed *in vacuo.* The product was recrystallised from methanol/chloroform to give 130g of product. Yield = 68%. ¹H-NMR (CDCl₃) δ 7.9-7.8 (m, 2H Ar-phthalimide), δ 7.75-7.65(m, 2H Ar-phthalimide), δ 7.3 (d, 1H, 6-H), δ 7.2 (S, 1H, 4-H), δ 6.5 (d, 1H, 7-H), δ 4.8 (s, 2H, β-CH₂), δ 3.9(s, 2H, Ar-CH₂) δ 3.0 (s, 3H, N-CH₃), δ 1.33 (s, 6H, (CH₃)₂). MALDI-TOF m/z = 332 (100%), M⁺ = 334 for C₂₁H₂₂N₂O₂.

### 8.2 2-[1E,3E)-5-(3,3-Dimethyl-5-sulfo-1,3-dihydro-2H-indol-2-ylidene)-1,3-pentadienyl]-5-[(1,3-dioxo-1,3-dihydro-2H-isoindol-2-yl)methyl]-1,3,3-trimethyl-3H-indolium

5-[1,3-Dioxo-1,3-dihydro-2*H*-isoindo)-2-yl)methy)]-1,2,3,3-tetramethyl-3H-indolium (20mg, 0.06mmol), 5-sulfo-2,3,3-trimethylindolenine (15.4mg, 0.06mmol) and malonaldehyde bis(phenylimine) monohydrochloride (17mg, 0.066mmol) was stirred in N,N'-dimethyl formamidine at 90°C. To this was added benzoic acid (8mg, 0.066mmol) and benzoic anhydride (15mg, 0.066mmol). The solution was heated for 3 hours before cooling to yield a dark blue solution. This was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 42.7 minutes. Yield: 23%. MALDI-TOF m/z = 609 (100%), M⁺ = 609 for C₃₅H₃₅N₃O₅S.

### 8.3 5-(Aminomethyl)-2-[1E,3E)-5-(3,3-dimethyl-5-sulfo-1,3-dihydro-2H-indol-2-ylidene)-1,3-pentadienyl]-1,3,3-trimethyl-3H-indolium

The phthalimide-protected product (see Section 8.2) was dissolved in ethanol. A fourfold excess of hydrazine hydrate was added upon which the solution became red. The reaction mixture was stirred at room temperature for 2 hours. The solvent was then removed under reduced pressure and the mauve solid was dissolved in distilled water. This was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 37 minutes. Yield: 40%. MALDI-TOF m/z 479 (100%) M⁺ = 479 for C₂₇H₃₃N₃O₃S.

### Example 9: 1-(5-Carboxypentyl)-2-[1E,3E)-5-(1,1-dimethyl-6-8-disuldo-1,3-dihydro-2H-benzo[e]indol-2-ylidene)-1,3-pentadienyl]-3,3-dimethyl-5-sulfo-3H-indolium (Compound 9)

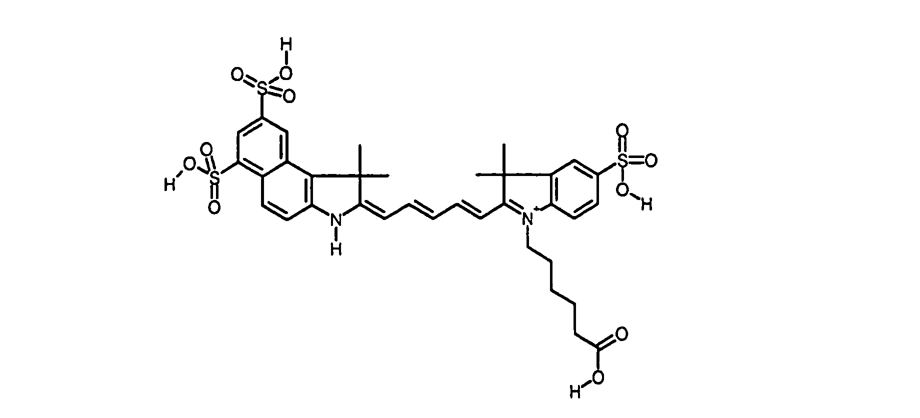

### 9.1 6-Hydrazinonaphthalene-1,3-disulfonate

2-Naphthylamine-5,7-disulfonic acid (10g, 0.033mol) was suspended in 50% hydrochloric acid (200ml). This was stirred at 0-5°C. Sodium nitrite (2.5g, 0.036 mol) in distilled water (15ml) was added dropwise. Stannous chloride (8.2g, 0.036mol in 10ml c. HCl) was added dropwise to the stirred mixture at < 5°C. The reaction mixture was stirred for 1 hour. The product was concentrated under reduced pressure and triturated with hot 2-propanol to yield a pale powder. ¹H NMR (*d*_{*6*}-DMSO)δ 8.72 (d, 1H, J = 9.2, 8-H), δ 8.1 (d, 1H, J = 1.2, 4-H), δ 7.95 (s, 1H, 2-H), δ 7.3 (s, 1H, 5-H), δ 7.15 (d, 1H, J = 9.2, 7-H). m/z = 318 (100%) M⁺ = 318 for C₁₀H₁₀N₂O₆S₂^{.}

### 9.2 1,1.2-Trimethyl-6.8-disulfo-benzo[e]indole

6-Hydrazinonaphthalene 1,3-disulfonate (6.2g, 0.02mol), methyl isopropyl ketone (6.4ml 0.06mol), potassium acetate (5.88g, 0.06mol) and glacial acetic acid (35ml) were refluxed at 135°C for 24hr. The acetic acid was removed *in vacuo* and the solid stirred with 2-propanol to yield a buff powder. ¹H NMR (D₂O), 8.8 (d, 1H, J = 7, 4-H), 8.7 (s, 1H, 9-H), 8.45 (s, 1H, 7-H), 7.9 (d, 1H, J = 7, 5-H), 2.4 (s, 3H, CH₃), 1.5 (s, 6H, (CH₃)₂) MALDI-TOF m/z = 369 M⁺ = 369 for C₁₅H₁₅NO₆S₂.

### 9.3 1-(5-Carboxypentyl)-2-[1E,3E)-5-(1,1-dimethyl-6-8-disulfo-1,3-dihydro-2H-benzo[e]indol-2-ylidene)-1,3-pentadienyl]-3,3-dimethyl-5-sulfo-3H-indolium

1,1,2-Trimethyl-6-8-disulfo-benzo[e]indole (20mg, 0.054mmol), 1-(5-Carboxypentyl)-5-sulfo-2,3,3-trimethylindole (19mg, 0.054mmol) and malonaldehyde bis(phenylimine) (15mg, 0.06mmol) were stirred in a 1:1:0.2 mixture of acetic acid, pyridine and acetic anhydride at 70°C for 4 hours to yield a dark blue solution. This solution was cooled. The dye was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 31 minutes (UV at 655nm). MALDI-TOF m/z = 758 (100%) M⁺= 758 for C₃₅H₃₈N₂O₁₁S₃.

### Example 10: 2-[1E,3E)-5-(3,3-Dimethyl-5-sulfo-1,3-dihydro-2H-indol-2-ylidene)-1,3-pentadienyl]-1-(6-hydrazino-6-oxohexyl)-3,3-dimethyl-5-sulfo-3H-indolium (Compound 10)

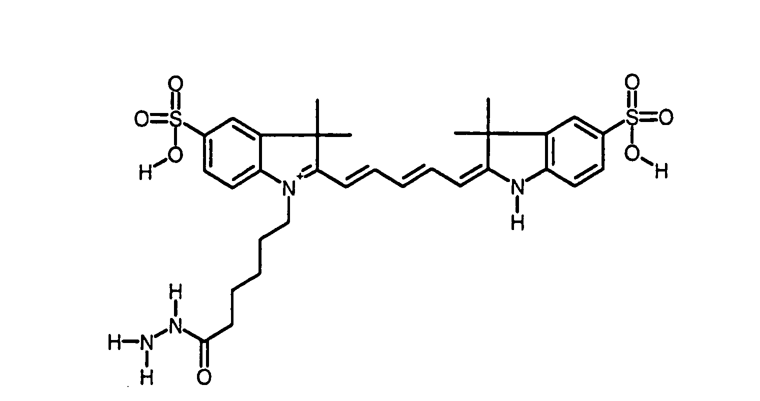

### 10.1 1-(5-Carboxypentyl)-2-[1E,3E)-5-(3,3-dimethyl-5-sulfo-1,3-dihydro-2H-indol-2-ylidene)-1,3-pentadienyl]-3,3-dimethyl-5-sulfo-3H-indolium

1-(5-Carboxy-pentyl-1,3,3-trimethyl-indolium-5-sulfonate (100mg, 0.28mmol) was dissolved in acetonitrile with 5-sulfo-2,3,3-trimethylindolenine (73mg, 0.28mmol) and malonaldehyde bis (phenylimine) (79mg, 0.31mmol). Benzoic acid (68mg, 56mmol) and benzoic anhydride (127mg, 0.56mmol) were added to the solution and the reaction mixture was stirred at 80°C for 2 hours. The dark blue solution was cooled and the solvent removed *in vacuo.* The remaining solid was dissolved in DMSO and the solution purified by reverse phase HPLC using a Rainin Dynamax 60Å C18 column at 10ml/min with a solvent gradient of 15% B for 5 minutes ramping from 15% to 50% B over 75minutes, where A = H₂O (0.1% Acetic acid) and B = acetonitrile (0.1 % acetic acid). The column retention time was 41 minutes (Vis. 650nm). MALDI-TOF m/z= 627.5 (100%) M⁺ = 630 for C₃₁H₃₈N₂O₈S₂.

### 10.2 2-[1E,3E)-5-(3,3-Dimethyl-5-sulfo-1,3-dihydro-2H-indol-2-ylidene)-1,3-pentadienyl]-1-(6-hydrazino-6-oxohexyl)-3,3-dimethyl-5-sulfo-3H-indolium

1-(5-Carboxypentyl)-2-[1*E*,3*E*)-5-(3,3-dimethyl-5-sulfo-1,3-dihydro-2*H*-indol-2-ylidene)-1,3-pentadienyl]-3,3-dimethyl-5-sulfo-3*H*-indolium (5mg, 7.9µmol) was dissolved in DMSO (200µl). To this solution was added diisopropylethylamine (5µl), benzotriazole-1-yl-oxy-tris-pyrrolidinophosphonium hexafluorophosphate (PyBOP) (4.4mg, 7.9µmol), N-hydroxy succinimide (1mg, 7.9µmol) and hydrazine hydrate (0.2µl, 2 equivalents). The bright red solution was stirred for 1 hour. The probe was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 18 minutes (UV at 650nm). MALDI-TOF m/z = 641 (100%). M⁺ = 644 for C₃₁H₄₀N₄O₇S₂.

### Example 11: pH Sensitive Cy3-Cy5 Energy Transfer Cassette (Compound 11).

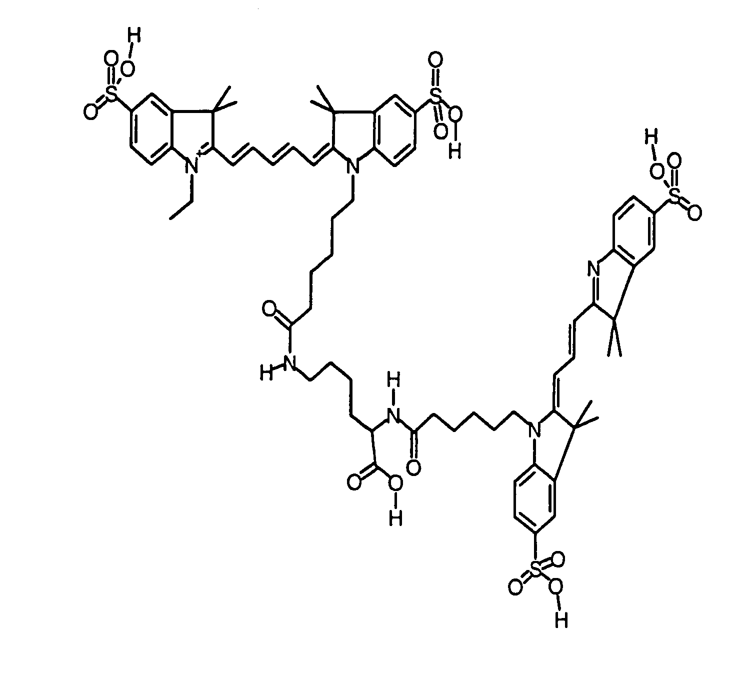

### 11.1 Cy5™:1-(5-Carboxypentyl)-2-[(1E,3E)-5-(1-ethyl-3,3-dimethyl-5-sulfo-1,3-dihydro-2H-indol-2-ylidene)-1,3-pentadienyl]-3,3-dimethyl-5-sulfo-3H-indolium

Cy5 was prepared according to the method described in *Bioconjugate Chemistry*, Volume 4, Number 2, page 105, (1993). 1-Ethyl-2,3,3-trimethyl-3*H*-indolium-5-sulfonate (4g, 0.015mol) and malonaldehyde bis (phenylimine) monohydrochloride (4.4g, 0.017mol) were refluxed in acetic acid (20ml) and acetic anhydride (20ml) for 4 hours. Pyridine (20ml) and 1-(5-carboxypentyl-1,3,3-trimethyl indoleninium-5-sulphonate (5.3g, 0.015mol) were then added to the solution and the reaction was heated for a further 3 hours. The product was washed with 2-propanol and purified by reverse phase chromatography. ¹H NMR (D₂O)δ 7.9 - 8.2 (m, 6H, 4-H, 4'-H, 6-H, 6'-H, β,β' protons of the bridge), δ 6.2 (m, 2H, α,α' protons of bridge), 7.5 (m, 2H, 7-H, 7-H'), δ 6.7 (t, 1H, J = 13, γ proton of bridge), δ 4.0 - 4.2 (m, 4H, α,α' -CH₂), 8 2.9 (t, 2H, J = 7, CH₂COOH), δ 1.4,-2 (m, 21H, 3 CH₂, 1 CH₃, 2 (CH₃)₂).

### 11.2 Cy5-lysine-fmoc

Cy5 (50mg, 0.072mmol) was dissolved in dimethyl sulphoxide (2ml). N, N-diisopropylethylamine (40ml), and *O*,-N-succinimidyl-N,N,N',N'-bis(tetramethylene)-uronium hexafluorophosphate (74mg, 0.18mmol) was added and the reaction stirred for 30 minutes. The reaction mixture was then quenched with diethyl ether, filtered and triturated with ethyl acetate to yield the product as a fine blue powder. The solid was then dissolved in dimethyl sulphoxide (2ml) and diisopropylethylamine (40ml) and *N*-(9-fluorenylmethoxycarbonyl)-L-lysine (40mg, 0.1mmol) were added. This was stirred at room temperature overnight. The product was purified by analytical reverse phase HPLC using a C₁₈ VYDAC column with a gradient of 20% to 40% B over 30 minutes, where A = water/0.1% trifluoroacetic acid and B = acetonitrile/0.1% trifluoroacetic acid. The column retention time for the product was 33 minutes. MALDI-TOF m/z = 1007 (100%) M⁺ = C₅₄H₆₃N₄O₁₁S₂.

### 11.3 Cy5-Lys

Cy5-Lys-fmoc (5mg, 4.9µmol) was dissolved in 4:1 DMSO/piperidine (200µl). This was stirred for 2 hours. The dye was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 26 minutes (UV at 655nm). MALDI-TOF m/z = 784 (100%) M⁺ = 786 for C₃₉H₅₄N₄O₉S₂.

### 11.4 pH Sensitive Cv3-Cv5 Energy Transfer Cassette

Cy5-Lys (1mg, 1.2µmol) and the NHS ester of Cy3.39 (Compound 3, Table 1) (0.89mg, 1.2µmol) were stirred in 200µl of DMSO and diisopropylethylamine (1µl) for 2 hours. The solution was purified by reverse phase analytical (1ml/min. flow) HPLC. The gradient was 15% B for 5 minutes and then 15% to 50% for 75 minutes, where eluent A is water (0.1% acetic acid) and eluent B is acetonitrile (0.1% acetic acid). The column retention time for the product was 33.5 minutes (UV at 655nm). MALDI-TOF m/z = 1376 (100%). M⁺ = 1374 for C₆₈H₈₈N₆O₁₆S₄.

### Example 12: Fluorescent Characteristics of pH Sensitive Cyanine Dyes.

Equimolar solutions of compound Cy3.39 and compounds 4-10 were prepared in a range of phosphate buffers (pH 5.85 - 9.86). The fluorescence and UV absorbance properties of each dye were observed at different pH. These properties are shown in Table 4.

**Table 4.**

| Spectral Properties of pH Sensitive Cyanine Dyes | | | | |
|---|---|---|---|---|
| Compound | λₘₐₓ pH 5.00 | λₘₐₓpH 10.00 | λ_{Em}(nm) | pKa |
| 3.39 | 550nm | 476nm | 565nm | 7.75 |
| 4 | 650nm | 515nm | 665nm | 6.4 |
| 5 | 650nm | 484nm | 665nm | 7.5 |
| 6 | 552nm | 433nm | 580nm | 9.8 |
| 7 | 640nm | 531nm | 670nm | 6.85 |
| 8 | 650nm | 531nm | 665nm | 7.25 |
| 9 | 655nm | 522nm | 680nm | 7.25 |
| 10 | 653nm | 501nm | 660nm | 7.5 |

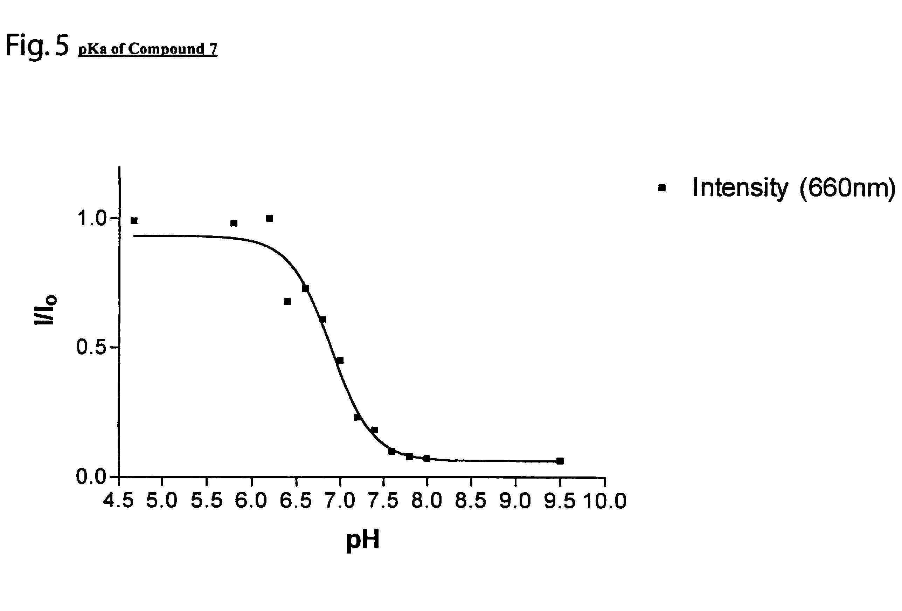

The pKa was measured as the ratio of emission intensity at the pH measured and the emission intensity for maximum emission, or I/Iₒ. This is illustrated in Figure 5 for Example 7 using a sigmoidal curve fit. The pKa can be calculated to be where I/Iₒ = 0.5 or where the fluorescent probe is 50% protonated.

### Example 13: Fluorescent Properties of a pH Sensitive Energy Transfer Probe

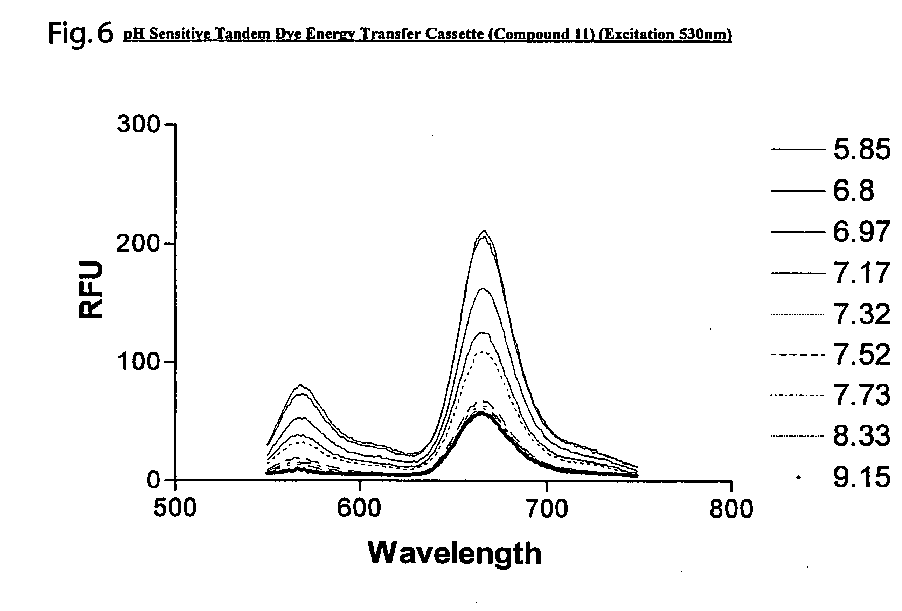

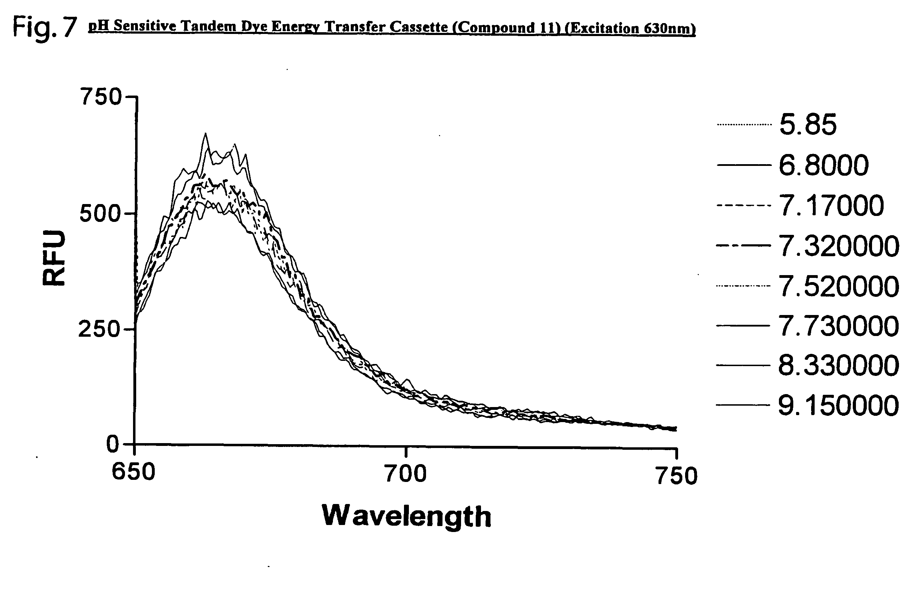

The fluorescent properties of a tandem dye energy transfer cassette consisting of a pH sensitive donor Cy3 and a fluorescent acceptor Cy5, held within a finite distance *via* a lysine linker have been investigated. Equimolar concentrations of the probe were made up in phosphate buffers of differing pH. Figure 6 illustrates the fluorescent spectra of the probe at different pH upon excitation at 530nm. It is observed that the fluorescent emission of the pH sensitive donor (Cy3, 560nm) decreases when the probe is within a less acidic or more alkaline environment. Consequently the efficiency of energy transfer from the Cy3 pH sensitive donor to the non-pH sensitive Cy5 acceptor dye (sensitised emission 660nm) also decreases over the physiological pH range of 5.8 - 9.2. Exciting this probe at the excitation wavelength of the acceptor (630nm) observes emission at 660nm with little change in intensity (±50nm) over physiological pH range 5.8 - 9.2 (Figure 7).

### Example 14: Cell Internalisation Assay Utilising pH Sensitive Cy5™ (Compound 4)

### 14.1 Methods

CHOM1 cells were seeded onto 3cm glass bottomed tissue culture coated dishes at approximately 50% confluency. The cells were grown in F12 Ham's media containing 10% foetal calf serum and 2mM glutamine. The cells were then incubated for 20mins on ice, washed twice in serum free F12 and then incubated with 0.1mg/ml of Compound 4 N-hydroxysuccinimidyl ester for 1.5 hours at 4°C. The cells were then washed with serum free media and analysed immediately using a Zeiss LSM410 confocal microscope x 63 oil objective, 633nm excitation and 665nm emission (before internalisation). The cells were then stimulated to internalise by constitutive endocytosis, by incubating them at 37°C for 1 hour. They were then analysed using identical confocal microscopy conditions (after intemalisation).

### 4.2 Results

A marked increase in cellular fluorescence was observed as the pH sensitive dye was internalised from the neutral surrounding media (approximately pH 7.4) into acidic vesicles (pH 5.5 -6.0) within the cell. Cells that were incubated in a similar manner with Cy3-NHS ester showed no concurrent increase in fluorescence with internalisation. The intemalisation of the dye into the cell was clarified by examining serial z-sections through the cell using the Zeiss confocal microscope.

All references cited herein are incorporated in their entirety by reference.

## Claims

1. Use as a pH indicator of a water soluble dye consisting of a cyanine having the structure: 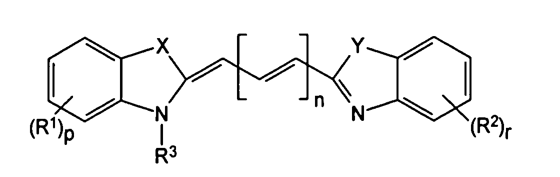 wherein:
X and Y are independently selected from >C(C₁ - C₄alkyl)₂, sulfur and oxygen;
R¹ and R² are independently selected from H, CH₂NH₂, SO₃⁻, phosphate, phosphonate, quaternary ammonium, (CH₂)_{q}COOH, NCS, CH₂NH-COR⁷, where R⁷ is C₁ - C₂₀ straight or branched alkyl and (CH₂)_{q}COOH where q is an integer from 0 - 10;
R³ is H or -L-P where L is selected from C₁ - C₂₀ straight or branched alkyl optionally containing 0, 1 or 2 unsaturated groups selected from alkenyl and alkynyl, and P is selected from a reactive group, H, C₁- C₂₀ straight or branched alkyl, SO₃⁻, NH₂, quaternary ammonium, CH₂NH-COR⁸, where R⁸ is C₁ - C₂₀ straight or branched alkyl, -(CH₂)ₘCOOH, NHR⁹ where R⁹ is C₁ - C₂₀ straight or branched alkyl;
n is an integer from 0 - 3; p and r are independently 0, 1, 2, 3 or 4 and where p and/or r is greater than 1, each R¹ and each R² may be different, and m is an integer from 1 - 10; and salts and protonated derivatives thereof.

2. A water soluble dye consisting of a cyanine having the structure: 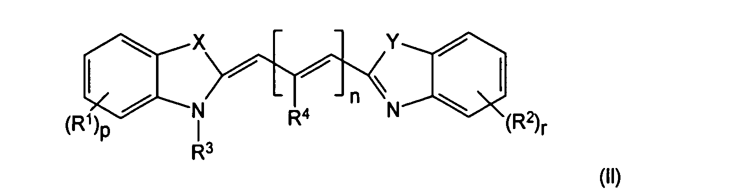 or 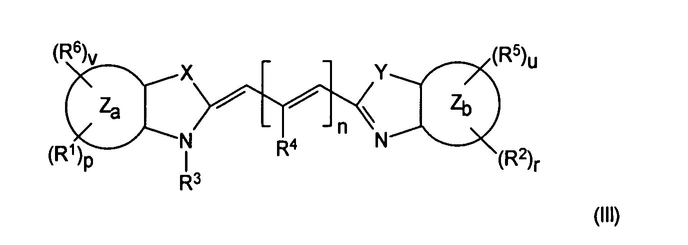 wherein:
X and Y are selected from >C(C₁ - C₄alkyl)₂, sulfur and oxygen;
Zₐ and Z_{b} each independently represent the atoms necessary to complete 1 or 2 fused aromatic rings having 6 carbon atoms, containing 0, 1 or 2 nitrogen atoms;
R¹, R², R⁵ and R⁶ are independently selected from H, CH₂NH₂, SO₃⁻, phosphate, phosphonate, quaternary ammonium, (CH₂)_{q}P, (CH₂)_{q}COOH, CH₂NH-COR⁷, where R⁷ is C₁ - C₂₀ straight or branched alkyl or (CH₂)ₘCOOH where q is an integer from 0 - 10; R³ is H or -L-P where L is selected from C₁ - C₂₀ straight or branched alkyl optionally containing 0, 1 or 2 unsaturated groups selected from alkenyl and alkynyl, and P is selected from a reactive group, H, SO₃⁻, NH₂, quaternary ammonium, CH₂NH-COR⁸ where R⁸ is C₁ - C₂₀ straight or branched alkyl, -(CH₂)ₘCOOH and NHR⁹ where R⁹ is C₁ - C₂₀ straight or branched alkyl;
R⁴ is H or (CH₂)ₘP;
n is an integer from 0 - 3; p, r, u and v are independently 0, 1, 2, 3 or 4 and where p, r, u and/or v is greater than 1, each R', R², R⁵ and R⁶ may be different, and m is an integer from 1 - 10;
wherein at least one of R¹, R², R³, R⁴, R⁵ and R⁶ is a substituent for making the dye covalently reactive with a component to be labelled; and salts and protonated derivatives thereof.

3. A dye according to claim 2 wherein said substituent is covalently reactive with an amine, hydroxy, aldehyde and/or sulfhydryl group on said component.

4. A dye according to claim 2 wherein reactive group P is selected from hydroxy succinimide ester, hydroxy sulfosuccinimide ester, anhydride, haloacetamide, maleimide, sulphonyl halide, phosphoramidite, acid halide, alkylimidate, hydrazide, carbodiimide, isothiocyanate, isocyanate, monochlorotriazine, dichlorotriazine, mono- or di-halogen substituted pyridine, mono- or di-halogen substituted diazine, aziridine, imido ester, hydrazine, azidonitrophenyl, azide, 3-(2-pyridyldithio)-propionamide, glyoxal, and aldehyde and groups reactive with amino, hydroxyl, aldehyde, phosphoryl, or sulphydryl groups.

5. A dye according to claim 2 wherein the reactive group P is selected from:
―NCS; 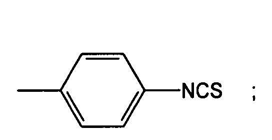
―(CH₂)ₛNCS ; ―NCO; 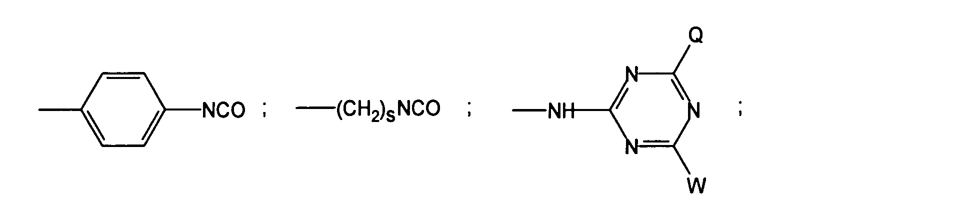 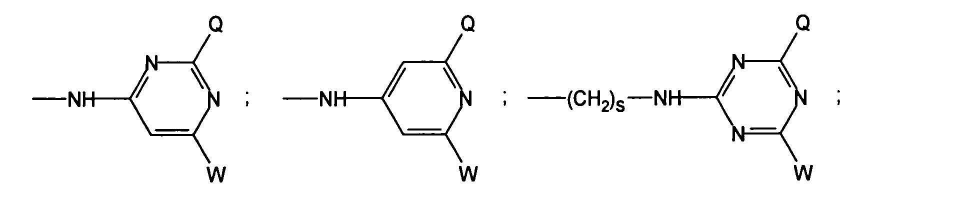 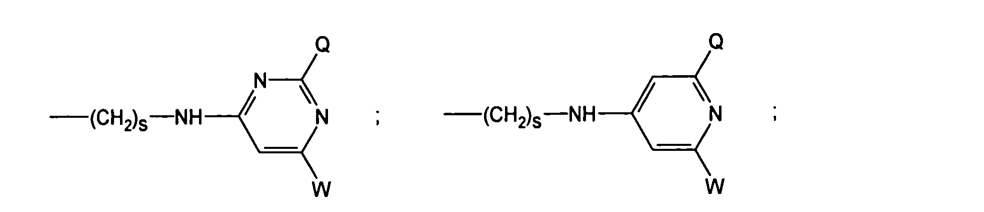 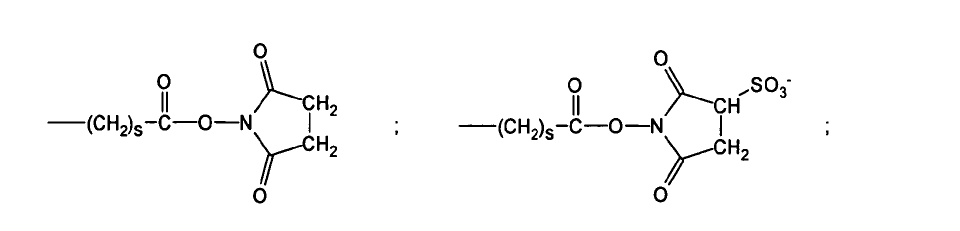 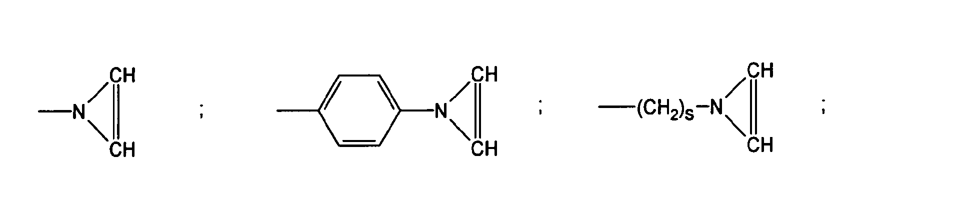 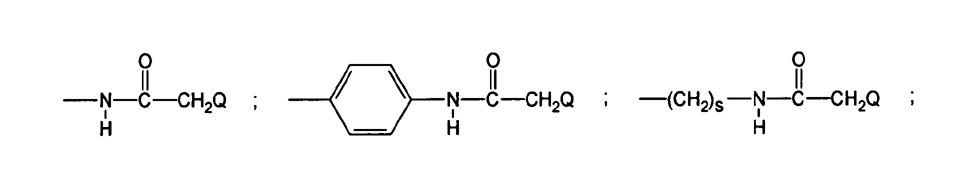 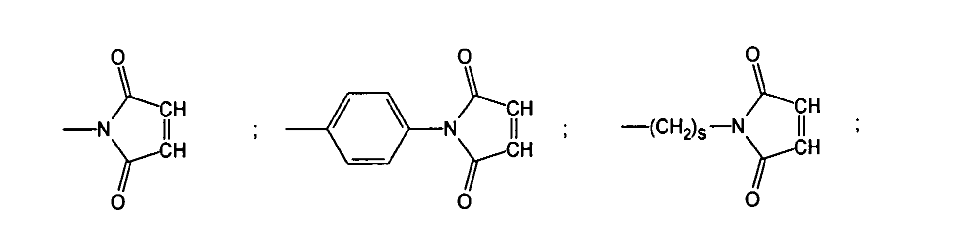 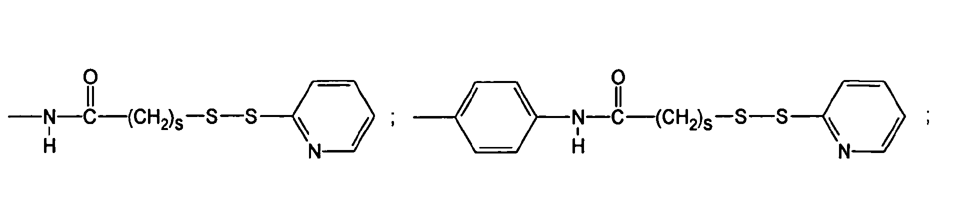 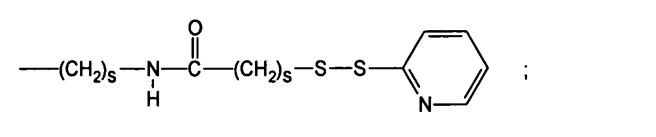
―N₃ ; 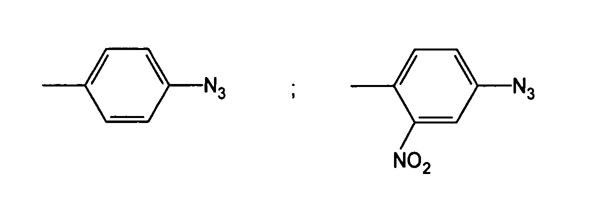
wherein at least one of Q or W is a leaving group and s is 0 or an integer from 1 - 8.

6. A component labelled with a dye according to any of claims 2 to 5 wherein said component is selected from the group consisting of antibody, protein, peptide, enzyme substrate, hormone, lymphokine, metabolite, receptor, antigen, hapten, lectin, avidin, streptavidin, toxin, carbohydrate, oligosaccharide, polysaccharide, nucleic acid, derivatized deoxy nucleic acid, DNA fragment, RNA fragment, derivatized DNA fragment, derivatized RNA fragment, drug, virus particle, bacterial particle, virus component, yeast component, blood cell, blood cell component, biological cell, noncellular blood component, bacteria, bacterial component, natural and synthetic lipid vesicle, and poison.

7. A component labelled with a dye according to any of claims 2 to 5 wherein said component is selected from the group consisting of consisting of environmental pollutant, polymer, polymer particle, glass particle, glass surface, plastic particle, plastic surface, polymer membrane, conductor and semiconductor.

8. A method of detecting acidic or basic conditions comprising mixing a compound according to any of claims 2 to 5 with a composition suspected of being acidic or basic and detecting fluorescence of said compound as an indication of said acidic or basic conditions.

9. The method according to claim 8 wherein said acidic or basic condition is an intracellular environment.

10. The method according to claim 8 wherein said detecting is accomplished with a fluorescence microscope, fluorimeter, fluorescence cell sorter, or fluorescence equipped plate reader.

11. A dye conjugate comprising a dye of formula: 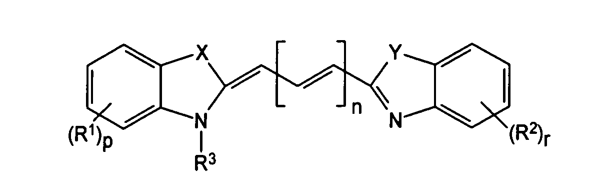 wherein:
X, Y, n, p and r are as hereinbefore defined;
either of R¹, R², or R³ is linked in an energy transfer relationship to a fluorescent dye through a linker group L; wherein L is selected from a straight or branched C₁₋₂₀alkyl chain, a C₂₋₂₀ monoether or polyether and a C₂₋₂₀ atom chain containing up to four secondary amide linkages; and remaining groups R¹, R² and R³ are as hereinbefore defined; and salts and protonated derivatives thereof.

12. A dye conjugate according to claim 11 wherein said conjugate contains a target bonding group, P capable of forming a covalent bond with a target material.

13. A dye conjugate according to claim 12 wherein P is selected from hydroxy-succinimidyl ester, hydroxy sulfosuccinimidyl ester, anhydride, haloacetamide, maleimide, sulphonyl halide, phosphoramidite, acid halide, alkylimidate, hydrazide, carbodiimide, isothiocyanate, isocyanate, monochlorotriazine, dichlorotriazine, mono- or di-halogen substituted pyridine, mono- or di-halogen substituted diazine, aziridine, imido ester, hydrazine, azidonitrophenyl, azide, 3-(2-pyridyldithio)-propionamide, glyoxal, and aldehyde and groups reactive with amino, hydroxyl, aldehyde, phosphoryl, or sulphydryl groups.

14. A dye conjugate according to claim 11 wherein said conjugate includes a functionality to facilitate cell permeability.

15. A ratiometric, concentration independent, method of measuring pH of an environment, comprising adding a dye conjugate according to any of claims 11 to 14 to said environment and measuring, in a ratiometric manner, the fluorescence intensity ratio.

16. Use of a dye according to any of claims 2 to 5 or a dye conjugate according to any of claims 11 to 14 for analysis or detection.

17. Use according to claim 16 wherein said detection is by optical means.
